# EUROPÄISCHE PATENTANMELDUNG

(11) **EP 4 707 802 A1**
(43) Veröffentlichungstag der Anmeldung: **11.03.2026**
(21) Anmeldenummer: 25198581.8
(22) Anmeldetag: 28.08.2025
(51) Int. Cl.: G01N 33/497

(54) **ANALYSEGERÄT ZUR KONZENTRATIONSMESSUNG MIT VENTIL UND FILTER**

(30) Priorität: 06.09.2024 DE 102024125707
(71) Anmelder: Dräger Safety AG & Co. KGaA, 23560 Lübeck (DE)
(72) Erfinder: Reier, Tobias, 23558 Lübeck (DE); Baesler, Malte, 23558 Lübeck (DE); Brunswick Franco, Luis, 23558 Lübeck (DE); da Silva Cirolini, Roberto, 23558 Lübeck (DE)

(57) **Zusammenfassung**

Die Erfindung betrifft ein Analysegerät (100), welches dazu ausgestaltet ist, ein Gasgemisch auf eine vorgegebene Substanz zu untersuchen. Eine röhrenförmige Eingabeeinheit (70) lässt sich mit einem Grundkörper verbinden. Ein Gasgemisch lässt sich in die Eingabeeinheit eingeben und fließt von einem Einlass zu einem Auslass. Eine Fluidführungseinheit (71) verbindet eine Ansaugöffnung (AO) in der Eingabeeinheit (70) mit einer Messkammer (3). Eine Ansaugeinheit (5) vermag eine Gasprobe (Gp) aus der Eingabeeinheit (70) anzusaugen und durch die Fluidführungseinheit (71) hindurch in die Messkammer (3) zu fördern. Ein Sensor vermag die Konzentration der Substanz in einer Gasprobe (Gp), die sich in der Messkammer (3) befindet, zu messen. Ein Ventil gibt wahlweise die Fluidführungseinheit (71) frei oder versperrt sie. Ein elektrostatisch aufgeladener und / oder mechanisch wirkender Filter (23) in der Fluidführungseinheit (71) befindet sich zwischen der Ansaugöffnung (AO) und dem Ventil.

## Beschreibung

Die Erfindung betrifft ein Analysegerät, welches ein Gasgemisch auf eine vorgegebene Substanz zu analysieren vermag und hierbei die Konzentration der Substanz im Gasgemisch zu messen vermag.

In einer Anwendung ist das Gasgemisch eine Atemprobe, die ein Proband ausgeatmet hat, und die Substanz ist Atemalkohol oder eine sonstige Substanz, die sich in einer Atemprobe eines Probanden nachweisen lässt. Bei dieser Anwendung soll geprüft werden, ob der Proband Alkohol oder die sonstige Substanz zu sich genommen hat und die Substanz daher in seinem Körper vorhanden ist oder nicht.

Der Erfindung liegt die Aufgabe zugrunde, ein Analysegerät bereitzustellen, welches die Konzentration einer Substanz in einem Gasgemisch zu messen vermag und auch bei einem längeren Einsatz eine höhere Zuverlässigkeit als bekannte Analysegeräte aufweist.

Die Aufgabe wird durch ein Analysegerät mit den Merkmalen des Anspruchs 1 gelöst. Vorteilhafte Ausgestaltungen sind in den Unteransprüchen angegeben.

Das erfindungsgemäße Analysegerät vermag ein Gasgemisch auf eine vorgegebene Substanz zu analysieren, insbesondere eine Atemprobe auf Atemalkohol.

Das Analysegerät umfasst einen Grundkörper und eine Eingabeeinheit. Die Eingabeeinheit ist mit dem Grundkörper verbunden oder lässt sich mit dem Grundkörper verbinden. In einer ersten Realisierungsform ist die Eingabeeinheit fest mit dem Grundkörper verbunden. In einer zweiten Realisierungsform umfasst die Eingabeeinheit ein Mundstück und eine Röhre, wobei das Mundstück lösbar mit der Röhre und die Röhre fest mit dem Grundkörper verbunden ist. In einer dritten Realisierungsform ist die gesamte Eingabeeinheit lösbar mit dem Grundkörper verbunden, lässt sich also als Ganzes wieder vom Grundkörper trennen. In vielen Fällen erleichtern die zweite und dritte Realisierungsform es besser, Anforderungen an die Hygiene einzuhalten.

Die Eingabeeinheit hat die Form einer Röhre, insbesondere die Form eines Zylinders oder eines Kegelstumpfs oder eines Prismas mit einer n-eckigen Querschnittsfläche, n >= 3. Die Eingabeeinheit weist einen Einlass, einen Auslass und eine Mantelfläche auf. Die Mantelfläche erstreckt sich zwischen dem Einlass und dem Auslass. In einer Ausgestaltung verjüngt sich die Eingabeeinheit vom Einlass zum Auslass hin. Ein zu analysierendes Gasgemisch lässt sich durch den Einlass hindurch in die Eingabeeinheit eingeben. Das eingegebene Gasgemisch fließt durch die Eingabeeinheit hindurch in Richtung des Auslasses. Das eingegebene Gasgemisch oder wenigstens ein nicht abgesaugter Teil des Gasgemischs fließt durch den Auslass hindurch wieder aus der Eingabeeinheit hinaus.

Im Inneren des Grundkörpers befindet sich eine Sensor-Anordnung. Die Sensor-Anordnung umfasst eine Messkammer und einen Sensor. Die Messkammer vermag eine zu untersuchende Gasprobe aufzunehmen.

In der Mantelfläche der Eingabeeinheit befindet sich eine Ansaugöffnung. Die Ansaugöffnung weist einen Abstand vom Einlass und einen Abstand vom Auslass auf. Eine Fluidführungseinheit verbindet eine Öffnung im Grundkörper mit der Messkammer. Wenn die Eingabeeinheit mit dem Grundkörper verbunden ist, so ist folgende Situation hergestellt: Die Fluidführungseinheit verbindet die Ansaugöffnung in der Eingabeeinheit mit der Messkammer. Eine Gasprobe kann aus der Eingabeeinheit durch diese Fluidführungseinheit hindurch in die Messkammer fließen. Die Fluidführungseinheit befindet sich im Inneren des Grundkörpers. Wenn die Eingabeeinheit nicht mit dem Grundkörper verbunden ist, so verbindet die Fluidführungseinheit eine Öffnung im Grundkörper mit der Messkammer.

Anmerkung: Unter einer Fluidführungseinheit wird ein Bauteil verstanden, welches ein Fluid, hier eine Gasprobe, entlang einer Trajektorie zu führen vermag, wobei diese Trajektorie durch die Konstruktion und Anordnung des Bauteils vorgegeben ist. Eine Röhre und ein Schlauch sind zwei Beispiele für eine Fluidführungseinheit.

Eine Ansaugeinheit im Inneren des Grundkörpers vermag folgende Schritte durchzuführen: Aus einem Gasgemisch, das durch die Eingabeeinheit hindurchfließt, saugt die Ansaugeinheit eine Gasprobe an, zweigt die Gasprobe also aus dem Gasgemisch ab. Die Gasprobe ist also ein Teil des durch die Eingabeeinheit fließenden Gasgemischs. Der Rest des Gasgemischs wird nicht abgesaugt, sondern bleibt in der Eingabeeinheit und fließt durch den Auslass hindurch aus der Eingabeeinheit hinaus. Die Ansaugeinheit fördert die Gasprobe durch die Ansaugöffnung und die Fluidführungseinheit hindurch in die Messkammer. Bevorzugt vermag die Ansaugeinheit zusätzlich die Messkammer auszuspülen.

Der Sensor der Sensor-Anordnung vermag die Konzentration (den Anteil) und / oder die Menge, z.B. die Masse, der gesuchten Substanz in einer Gasprobe zu messen, während diese Gasprobe sich in der Messkammer befindet. In der Regel misst der Sensor eine Detektionsgröße, die mit der Konzentration und / oder der Menge der Substanz in der Gasprobe korreliert. Eine signalverarbeitende Auswertungseinheit der Sensor-Anordnung wendet einen vorgegebenen funktionalen Zusammenhang auf die gemessene Detektionsgröße (genauer gesagt: auf wenigstens einen gemessenen Wert der Detektionsgröße) an und leitet dadurch die Konzentration und / oder die Menge der Substanz in der Gasprobe her. Optional hängt der funktionale Zusammenhang von mehreren gemessenen Größen ab.

Anmerkung: Die Formulierung wird verwendet, dass ein Sensor eine physikalische Größe zu messen vermag, beispielsweise die Konzentration einer Substanz in einer Gasprobe. Diese Formulierung bedeutet, dass der Sensor direkt die physikalische Größe zu messen vermag oder mindestens eine andere Größe, die mit der zu messenden Größe korreliert. Die oder eine gemessene andere Größe oder die Kombination der gemessenen anderen Größen zusammen sind daher ein Maß für die zu messende physikalische Größe. Die Messung liefert mindestens einen Wert für die gesuchte physikalische Größe.

Bevorzugt umfasst das Analysegerät eine eigene Ausgabeeinheit. Das Analysegerät gibt auf dieser Ausgabeeinheit die gemessene Konzentration der Substanz in visueller und / oder einer anderen von einem Menschen wahrnehmbaren Form aus. Optional gibt das Analysegerät auf der Ausgabeeinheit einen Alarm aus, wenn die Substanz-Konzentration außerhalb eines vorgegebenen Wertebereichs liegt.

Ein Ventil im Inneren des Grundkörpers lässt sich in eine verschließende Endposition und in eine freigebende Endposition überführen. In der verschließenden Endposition verschließt das Ventil die Fluidführungseinheit, so dass keine Fluidverbindung zwischen der Messkammer einerseits und der Ansaugöffnung in der Eingabeeinheit oder der Öffnung im Grundkörper und damit einer Umgebung des Analysegeräts andererseits hergestellt ist. In der freigebenden Endposition gibt das Ventil die Fluidführungseinheit frei, so dass eine Gasprobe aus der Eingabeeinheit durch die Fluidführungseinheit hindurch in die Messkammer fließen kann. Idealerweise trennt das Ventil in der verschließenden Endposition die Messkammer vollständig fluiddicht von der Umgebung und von der Eingabeeinheit.

In der Fluidführungseinheit ist ein Filter angeordnet. Der Filter befindet sich zwischen der oben erwähnten Öffnung im Grundkörper und der Messkammer. Dann, wenn die Eingabeeinheit mit dem Grundkörper verbunden ist, befindet der Filter sich zwischen der Ansaugöffnung und dem Ventil. Der Filter vermag aus einer Gasprobe, die durch die Fluidführungseinheit und damit durch den Filter hindurch in Richtung der Messkammer fließt, Partikel herauszufiltern. Mit der Formulierung, dass Partikel herausgefiltert werden, ist gemeint, dass die Gasprobe flussaufwärts vom Filter die Partikel aufweist oder aufweisen kann, dank des Filters aber flussabwärts vom Filter idealerweise keine Partikel mehr aufweist. Der Filter kann insbesondere mechanisch wirken und / oder elektrostatisch aufgeladen sein und dadurch Partikel anziehen und binden.

Anmerkung: In der Regel umfasst der Filter das eigentliche Filterelement und eine Halterung für das Filterelement. Die Gasprobe fließt durch das Filterelement hindurch. Mit der Formulierung, dass der Filter mechanisch wirkt und / oder elektrostatisch aufgeladen ist, ist gemeint, dass das Filterelement diese Eigenschaften aufweist.

Erfindungsgemäß lässt sich ein zu analysierendes Gasgemisch durch den Einlass hindurch in die Eingabeeinheit eingeben, und das eingegebene Gasgemisch fließt durch die Eingabeeinheit hindurch in Richtung des Auslasses. Derjenige Anteil des Gasgemischs, der nicht als Gasprobe abgezweigt und in die Messkammer angesaugt wird, fließt durch den Auslass hindurch in die Umgebung. Dieses Merkmal ist insbesondere dann von Vorteil, wenn ein Proband das Gasgemisch in die Eingabeeinheit eingibt. Die Gefahr wird reduziert, dass das vom Probanden eingegebene Gasgemisch einer Person, die das Analysegerät hält, ins Gesicht fließt, wobei diese Person der Proband selbst oder eine andere Person, die das Analysegerät hält, sein kann. Vielmehr lässt sich die Eingabeeinheit so positionieren, dass das Gasgemisch in eine gewünschte Richtung fließt. Nicht erforderlich ist es, in der Eingabeeinheit ein Rückhalteelement vorzusehen und / oder die Fließrichtung des Gasgemischs durch die Eingabeeinheit zu verändern.

Die Ansaugeinheit saugt die Gasprobe aus dem Gasgemisch, das durch die Eingabeeinheit hindurchfließt, ab. Dieses Absaugen lässt sich zeitlich steuern, insbesondere indem die Ansaugeinheit entsprechend angesteuert wird. Dadurch wird es möglich, aus dem Gasgemisch einen gewünschten Anteil abzusaugen. Insbesondere wird es möglich, eine bestimmte Menge und / oder einen bestimmten Teil des Gasgemischs anzusaugen, und zwar relativ unabhängig davon, welches Volumen das in die Eingabeeinheit eingegebene Gasgemisch aufweist oder mit welchem Druck oder welchem Volumenfluss dieses Gasgemisch eingegeben wurde oder wird. Weiterhin wird es möglich, das Absaugen in einer bestimmten Zeitspanne durchzuführen, während das Gasgemisch in die Eingabeeinheit eingegeben wird.

Falls das eingegebene Gasgemisch eine Atemprobe eines Probanden ist, so ermöglicht das Ventil es in vielen Fällen, dass die abgesaugte Gasprobe ausgeatmete Luft aus mindestens einer Region des Atemsystems des Probanden aufweist, aber keine ausgeatmete Luft aus mindestens einer anderen Region des Atemsystems enthält. Beispielsweise wird ermöglicht, dass die Gasprobe ausgeatmete Luft aus den oberen Atemwegen und / oder aus der Lunge aufweist, aber nicht aus dem Mundbereich. Dieses Merkmal erleichtert es, zuverlässig den Gehalt von Atemalkohol in der Atemprobe zu messen und dadurch insbesondere zu ermitteln, ob sich im Blut des Probanden Alkohol befindet oder nicht.

Erfindungsgemäß vermag das Ventil in der verschließenden Endposition die Fluidführungseinheit fluiddicht zu verschließen und damit die Messkammer fluiddicht von der Umgebung zu trennen. Dadurch wird einerseits die Gefahr verringert, dass ein Bestandteil eines Sensors verdunstet. Diese Gefahr ist insbesondere dann gegeben, wenn der Sensor ein elektrochemischer Sensor ist. Die Gefahr der Verdunstung würde ohne ein Ventil insbesondere dann auftreten, wenn das Analysegerät längere Zeit gelagert wird und daher die Messkammer über die Fluidführungseinheit längere Zeit dauerhaft in einer Fluidverbindung mit der Umgebung steht. Andererseits verringert das verschlossene Ventil die Gefahr, dass Partikel und störende Substanzen, insbesondere Störgase, von außen durch die Fluidführungseinheit hindurch in die Messkammer gelangen. Solche Partikel und Substanzen können zu einer Fehlfunktion des Analysegeräts führen.

In Verbindung mit einer Eingabeeinheit, die sich abnehmen lässt, erzielt die Verwendung des Ventils insbesondere folgenden Vorteil: Das Ventil erspart die Notwendigkeit, die Fluidführungseinheit mit einem Verschluss zu verschließen. Ein solcher Verschluss müsste abgenommen werden, bevor die Eingabeeinheit mit dem Grundkörper verbunden wird. Falls die Eingabeeinheit dauerhaft mit dem Grundkörper verbunden ist, so müsste der Verschluss sowohl auf den Einlass als auch auf den Auslass aufgesetzt werden und vor einer Benutzung entfernt werden. Dies ist aufwendig. Der Vorgang, den Verschluss zu entfernen, erfordert in der Regel eine Handlung eines Benutzers.

Erfindungsgemäß befindet sich ein Filter in der Fluidführungseinheit und dort zwischen der Ansaugöffnung und dem Ventil. Der Filter an dieser Stelle bewirkt insbesondere folgendes, falls das Ventil sich in der freigebenden Endposition oder in einer freigebenden Zwischenposition befindet und eine Gasprobe durch die Fluidführungseinheit hindurch angesaugt wird: Die angesaugte Gasprobe fließt zunächst durch den Filter und dann durch das Ventil hindurch - genauer gesagt: durch ein Filterelement des Filters. Der Filter filtert aus der Gasprobe idealerweise alle Partikel heraus, die eine bestimmte Eigenschaft aufweisen, beispielsweise größer als eine vorgegebene obere Schranke sind, in der Praxis wenigstens einen großen Anteil dieser Partikel. Die Konstruktion des Filters legt fest, welche Partikel herausgefiltert werden. Im Falle eines elektrostatisch aufgeladenen Filters scheiden Partikel mit dieser vorgegebenen Eigenschaft sich am Filter ab, also beispielsweise alle ausreichend großen Partikel. "Alle Partikel" ist eine ideale Ausgestaltung, die sich in der Realität meist nur näherungsweise erzielen lässt.

Dank des Herausfilterns wird die Gefahr verringert, dass folgendes unerwünschte Ereignis auftritt: Partikel fließen mitsamt der Gasprobe zum Ventil und setzen sich auf einem Ventilkörper und / oder auf einem Ventilkörper-Sitz oder einem sonstigen Bestandteil des Ventils fest oder fließen durch das Ventil hindurch und gelangen in die Messkammer. Partikel auf dem Ventilkörper oder auf dem Ventilkörper-Sitz können dazu führen, dass das Ventil auch in der verschließenden Endposition die Fluidführungseinheit nicht vollständig fluiddicht verschließt. Die Gefahr bestünde dann, dass die Messkammer dauerhaft in einer Fluidverbindung mit der Umgebung steht, also auch dann, wenn das Ventil in der verschließenden Endposition ist. Partikel in der Messkammer können zu fehlerhaften Analyseergebnissen führen.

Erfindungsgemäß befindet sich der Filter in der Fluidverbindung zwischen der Ansaugöffnung und der Messkammer. Dieses Merkmal ermöglicht es, die Vorteile des Filters zu erzielen, ohne dass notwendigerweise in der Eingabeeinheit ein Filter vorhanden sein muss. Durch die Eingabeeinheit fließt das gesamte eingegebene Gasgemisch, durch die Fluidführungseinheit nur der abgezweigte Teil des Gasgemischs. Durch die Eingabeeinheit fließt also mehr Gas als durch die Fluidführungseinheit, bevorzugt mindestens doppelt so viel, besonders bevorzugt mindestens fünfmal so viel, insbesondere mindestens zehnmal so viel. Ein Filter in der Eingabeeinheit würde daher stärker durch Partikel sowie mechanisch belastet als der erfindungsgemäße Filter in der Fluidführungseinheit. Außerdem setzt ein Filter in der Eingabeeinheit einem durchfließenden Gasgemisch zwangsläufig einen pneumatischen Widerstand entgegen, und das Gasgemisch müsste mit einem größeren Druck eingegeben werden. Beispielsweise müsste ein Proband stärker in die Eingabeeinheit hineinblasen. Außerdem würde ein Filter in der Eingabeeinheit in vielen Fällen den pneumatischen Widerstand der Eingabeeinheit erhöhen, verglichen mit einer Eingabeeinheit ohne Filter, was zu einem unerwünschten Rückstau des Gasgemischs in der Eingabeeinheit führen könnte. Dies wiederum könnte bewirken, dass ein Teil des Gasgemischs dem Probanden ins Gesicht fließt. Dies ist unerwünscht.

In einer Ausgestaltung ist der Filter elektrostatisch aufgeladen und scheidet dadurch Partikel ab, welche mit der angesaugten Gasprobe durch die Fluidführungseinheit fließen. Die Partikel fließen an mindestens einer geladenen Elektrode, bevorzugt an mehreren geladenen Elektroden, vorbei und werden dadurch ionisiert. Ein elektrisches Feld bewirkt, dass die ionisierten Partikel zu einer entgegengesetzt geladenen Sammelelektrode gezogen werden und sich an der Sammelelektrode ansammeln. In einer Realisierungsform eines elektrostatisch aufgeladenen Filters umfasst der Filter ein Faservlies. Die Gasprobe fließt durch das Faservlies hindurch. Besonders bevorzugt wird ein Melt-Blown-Faservlies als Faservlies verwendet.

In einer Ausgestaltung wirkt der Filter mechanisch. Der Filter vermag aus einer Gasprobe Partikel herauszufiltern, idealerweise alle Partikel, wenn diese Partikel größer sind als eine durch die Konstruktion des Filters vorgegebene obere Schranke. In einer Realisierungsform funktioniert der mechanische Filter wie ein Sieb (Oberflächenfilter).

In einer anderen Realisierungsform umfasst der Filter mehrere (mindestens zwei, in einer Ausgestaltung mindestens drei) Schichten und / oder ist als ein poröser Körper aufgebaut. Bevorzugt umfasst jede Schicht jeweils mehrere Fasern, und die Anordnung der Fasern bestimmt die Position und die Größe jeder Pore. Nicht erforderlich ist, dass die Poren ein regelmäßiges Muster bilden. Die Fasern sind bevorzugt so angeordnet, dass eine einzelne Pore einen Partikel nur dann durchlässt, wenn die Partikelgröße kleiner ist als eine vorgegebene obere Schranke. Die Öffnungen in verschiedenen Schichten, also die Poren, sind wie folgt versetzt zueinander angeordnet: Ein Partikel, das als Teil der Gasprobe durch den Filter fließt, kann nicht auf einem geraden Weg durch den Filter hindurch gelangen, sondern nur dann, wenn der Partikel mindestens einmal, bevorzugt mehrmals seine Richtung ändert, also abgelenkt (umgelenkt) wird. Häufig bleibt der Partikel bei einer solchen Richtungsänderung am Filter haften. Bei einem solchen Tiefenfilter können die Öffnungen (Poren) größer sein als bei einem Sieb (Oberflächenfilter), ohne dass größere Partikel den Filter passieren können.

In vielen Fällen weist ein Tiefenfilter bei gleicher Filterwirkung einen geringeren pneumatischen Widerstand auf als ein Oberflächenfilter, also ein Filter, der wie ein Sieb funktioniert. Außerdem vermag ein Tiefenfilter oft wesentlich mehr Partikel an sich zu binden wie ein Oberflächenfilter, bis der pneumatische Widerstand so groß wird, dass der Filter ausgetauscht werden muss. Dieses Merkmal führt dazu, dass sich der Tiefenfilter oft länger verwenden lässt als ein Oberflächenfilter.

Die beiden Ausgestaltungen, nämlich elektrostatisch aufgeladener Filter und mechanischer Filter, lassen sich miteinander kombinieren. Dank dieser Kombination filtert ein elektrostatisch aufgeladener Filter auch dann noch Partikel heraus, wenn der elektrostatische Filter entladen ist, was häufig nach einer längeren Einsatzdauer passiert.

Insbesondere aus folgendem Grund ist es von Vorteil, wenn der Filter einen relativ geringen pneumatischen Widerstand aufweist, was insbesondere durch einen Filter erzielt wird, der als Tiefenfilter ausgestaltet und / oder elektrostatisch aufgeladen ist: Bei einem sonst gleichbleibenden Ansaugvorgang wird die Gasprobe bei einem geringen pneumatischen Widerstand rascher in die Messkammer gesogen als bei einem hohen pneumatischen Widerstand. Eine hohe Geschwindigkeit ist beispielsweise dann vorteilhaft, wenn das eingegebene Gasgemisch eine Atemprobe eines Probanden ist: Eine hohe Geschwindigkeit vergrößert die Zuverlässigkeit, dass die Gasprobe tatsächlich aus einem bestimmten gewünschten Teil des Atemsystems des Probanden stammt, beispielsweise aus der Lunge oder den oberen Atemwegen.

In einer Ausgestaltung filtert der Filter aus der durchfließenden Gasprobe alle Partikel heraus, die einen maximalen Durchmesser haben, der kleiner als oder höchstens genauso groß wie eine obere Schranke ist. Diese obere Schranke liegt bevorzugt zwischen 1 µm und 10 µm, besonders bevorzugt zwischen 1 µm und 3 µm, und beträgt insbesondere 2 µm.

Bevorzugt lassen die gesamte Eingabeeinheit oder wenigstens ein Mundstück sich lösbar mit dem Grundkörper verbinden und auch wieder vom Grundkörper trennen. Diese Ausgestaltung erleichtert es, hygienische Anforderungen einzuhalten. Beispielsweise gibt ein Proband eine Atemprobe in die Eingabeeinheit ein, und die Eingabeeinheit oder wenigstens das Mundstück wird einmal für diese eine Abgabe einer Atemprobe verwendet und anschließend entsorgt. Derselbe Grundkörper lässt sich hingegen dafür verwenden, um nacheinander mehrere Gasproben zu analysieren. Erfindungsgemäß befindet der Filter sich im Inneren des Grundkörpers und nicht in der Eingabeeinheit. Daher lässt sich auch der Filter mehrfach wiederverwenden. Verglichen mit einem Filter in der Eingabeeinheit reduziert dies den Materialverbrauch. Außerdem schützt der Filter das Ventil auch dann vor Verunreinigungen von außen, wenn der Grundkörper, also das Analysegerät ohne eine Eingabeeinheit, gelagert wird. Dank des Filters ist es seltener erforderlich, einen Verschluss auf eine Öffnung im Grundkörper aufzusetzen, wobei die Fluidführungseinheit diese Öffnung mit der Messkammer verbindet.

Erfindungsgemäß saugt die Ansaugeinheit aus dem Gasgemisch, das durch die Eingabeeinheit hindurchfließt, eine Gasprobe ab und fördert sie in die Messkammer. Die Gasprobe hat bevorzugt ein Volumen von weniger als 10% des Volumens des Gasgemischs, das durch die Eingabeeinheit hindurchfließt, besonders bevorzugt weniger als 1 %, insbesondere weniger als 0,1 %. Dadurch wird der Filter besonders wenig belastet und setzt sich besonders wenig mit Partikeln zu, verglichen mit einer größeren abgesaugten Gasprobe.

Bevorzugt umfasst das Ventil einen Ventilkörper und einen Ventilkörper-Sitz. Wenn das Ventil in der verschließenden Endposition ist, liegt der Ventilkörper am Ventilkörper-Sitz an, idealerweise fluiddicht. Wenn das Ventil in der freigebenden Endposition ist, tritt ein Abstand zwischen dem Ventilkörper und dem Ventilkörper-Sitz auf.

In einer Ausgestaltung umfasst das Analysegerät ein mechanisches Verbindungselement. Das Verbindungselement verbindet den Ventilkörper des Ventils mechanisch mit der Ansaugeinheit. Wenn die Ansaugeinheit eine Gasprobe ansaugt und in die Messkammer fördert, bewegt das mechanische Verbindungselement das Ventil aus der einen Endposition in die andere Endposition. Bei dieser Bewegung wird der Ventilkörper relativ zum Ventilkörper-Sitz bewegt. Bevorzugt überführt die Ansaugeinheit das Ventil vor dem Ansaugen einer Gasprobe aus der verschließenden in die freigebende Endposition und beim Ansaugen wieder aus der freigebenden in die verschließende Endposition. Die beiden Vorgänge, die Gasprobe anzusaugen und das Ventil zu öffnen, sind also miteinander synchronisiert und überlappen sich zeitlich. Nach dem Ansaugen ist die Fluidführungseinheit dann wieder verschlossen. Die umgekehrte Realisierungsform ist ebenfalls möglich. Bevorzugt vermag das Verbindungselement im Grundkörper zwei lineare Bewegungen in zwei zueinander entgegengesetzte Richtungen auszuführen.

Dank des mechanischen Verbindungselements reicht es aus, dass das Analysegerät einen einzigen Antrieb umfasst. Dieser eine Antrieb vermag einerseits die Ansaugeinheit zu bewegen und dadurch eine Gasprobe in die Messkammer zu fördern. Andererseits vermag dieser Antrieb das Ventil aus der einen Endposition in die andere Endposition zu überführen. Dank des Verbindungselements ist es nicht erforderlich, zwei verschiedene Antriebe vorzusehen. Dies spart Bauraum ein.

Diese Ausgestaltung ermöglicht folgende Betriebsweise: Wenn keine Gasprobe angesaugt wird, ist das Ventil in der verschließenden Endposition. Um eine Gasprobe anzusaugen, werden nacheinander die folgenden beiden Schritte durchgeführt: Im ersten Schritt fördert die Ansaugeinheit Gas aus einem Reservoir in die Messkammer und spült dadurch die Messkammer aus. Bevorzugt befindet das Reservoir sich im Inneren des Grundkörpers. Beim Ausspülen wird Gas aus der Messkammer durch die Fluidführungseinheit hindurch aus dem Gehäuse des Analysegeräts heraus und in die Eingabeeinheit hinein gefördert. Gleichzeitig oder wenigstens zeitlich überlappend wird mit Hilfe des Verbindungselements der Ventilkörper vom Ventilkörper-Sitz wegbewegt, und das Ventil wird in die freigebende Endposition überführt. Das Gas aus der Messkammer wird aus dem Analysegerät hinausgespült. Im zweiten Schritt saugt die Ansaugeinheit eine Gasprobe aus der Eingabeeinheit an. Die Gasprobe fließt durch die Fluidführungseinheit hindurch in die Messkammer. Gleichzeitig oder wenigstens zeitlich überlappend wird mithilfe des Verbindungselements der Ventilkörper wieder auf den Ventilkörper-Sitz zu bewegt, und das Ventil wird wieder in die verschließende Endposition überführt. Dadurch verschließt das Ventil so lange wie möglich die Fluidführungseinheit, und die Messkammer steht nur so lange wie nötig in einer Fluidverbindung mit der Umgebung. Das verschlossene Ventil und der Filter trennen die Messkammer auch dann von der Umgebung, wenn die Eingabeeinheit vom Grundkörper getrennt ist.

In einer bevorzugten Ausgestaltung umfasst die Ansaugeinheit eine Kammer mit einem veränderbaren Volumen, insbesondere einen Balg oder eine Kolben-Zylinder-Einheit. Die Messkammer befindet sich zwischen der Kammer der Ansaugeinheit und der Fluidführungseinheit. Die Kammer der Ansaugeinheit befindet sich in einer Fluidverbindung mit der Messkammer. Die Kammer stellt das oben beschriebene Reservoir bereit. Wird das Volumen der Kammer vergrößert, so wird in der Kammer der Ansaugeinheit, dadurch in der Messkammer und dadurch in der Fluidführungseinheit ein Unterdruck erzeugt, und aufgrund des entstehenden Unterdrucks wird eine Gasprobe durch die Fluidführungseinheit hindurch in die Messkammer angesaugt. Wird das Volumen der Kammer verringert, so wird in der Kammer der Ansaugeinheit und dadurch in der Messkammer ein Überdruck erzeugt, und dadurch wird die Messkammer ausgespült.

Bevorzugt verbindet das mechanische Verbindungselement die Ansaugeinheit wie folgt mit dem Ventil: Wenn die Kammer in den Zustand mit minimalem Volumen überführt wird, so wird das Ventil in die freigebende Endposition überführt. Wenn die Kammer umgekehrt in den Zustand mit maximalem Volumen überführt wird, so wird das Ventil in die verschließende Endposition überführt.

Bevorzugt umfasst das Analysegerät eine Heizung. Die Heizung vermag ein Segment der Fluidführungseinheit zu erwärmen. Der Filter befindet sich im erwärmbaren Segment. Die Heizung kann einen Draht umfassen, der elektrisch leitend ist und sich erwärmt, wenn er von Strom durchflossen wird. In einer anderen Realisierungsform erwärmt die Heizung das Segment berührungslos. Eine Strahlungsquelle emittiert elektromagnetische Strahlung, insbesondere Infrarotstrahlung, und die emittierte Strahlung erwärmt das Segment.

In vielen Fällen umfasst das Gasgemisch, das durch die Eingabeeinheit hindurchfließt, Flüssigkeitströpfchen und / oder Dampf. Dies gilt insbesondere dann, wenn das Gasgemisch eine Atemprobe ist, die ein Proband abgibt. Die abgesaugte Gasprobe umfasst daher meist ebenfalls Flüssigkeitströpfchen und / oder Dampf. Die Gefahr besteht, dass auf einem Filterelement des Filters in der Fluidführungseinheit Flüssigkeit kondensiert und beispielsweise vom Filter abperlt. Diese kondensierte Flüssigkeit könnte zu einem größeren pneumatischen Widerstand führen und / oder das Filterelement beschädigen und / oder ein Messergebnis der Sensor-Anordnung verfälschen. Insbesondere könnte Flüssigkeit an einer Innenwand der Fluidführungseinheit oder auf einem optischen Element der Sensor-Anordnung kondensieren.

Bekanntlich vermag ein Gasgemisch bei sonst gleichbleibenden Bedingungen umso mehr Flüssigkeit aufzunehmen, je größer die Temperatur des Gasgemischs ist. Die Heizung reduziert daher die Gefahr, dass auf dem Filter Flüssigkeit kondensiert. Weil der Filter sich im erwärmten Segment befindet, ist die Gefahr der Kondensation geringer, als wenn die Heizung sich an einer anderen Stelle befinden würde.

Die röhrenförmige Eingabeeinheit erstreckt sich entlang einer Längsachse. Bevorzugt erstreckt sich die Fluidführungseinheit ebenfalls entlang einer Längsachse. Bevorzugt schließen diese beiden Längsachsen zwischen sich einen Winkel von mindestens 60 Grad ein. Besonders bevorzugt stehen die beiden Längsachsen senkrecht aufeinander. Diese Ausgestaltung erleichtert es, dass bei einer Benutzung des Analysegeräts die Längsachse der Eingabeeinheit annähernd horizontal und die Längsachse der Fluidführungseinheit annähernd vertikal angeordnet sind. Der Grundkörper lässt sich bequem in einer Hand halten. Die Richtung, in der das Gasgemisch die Eingabeeinheit verlässt, lässt sich leicht festlegen.

In einer Ausgestaltung umfasst das Analysegerät einen Drucksensor. Der Drucksensor vermag mindestens einmal, bevorzugt wiederholt, den Druck an einer ersten Messposition zu messen, insbesondere mit einer festen Abtastfrequenz. Die erste Messposition befindet sich flussabwärts vom Filter und außerhalb der Eingabeeinheit. In einer ersten Realisierungsform befindet sich die erste Messposition in oder an der Fluidführungseinheit und dort zwischen dem Filter und der Messkammer, in einer zweiten Realisierungsform in der Messkammer oder an einer Wand der Messkammer und in einer dritten Realisierungsform zwischen der Messkammer und der Ansaugeinheit. Die erste Messposition befindet sich also im Inneren des Grundkörpers und außerhalb der Eingabeeinheit, und daher hängt häufig der Druck an der ersten Messposition relativ wenig von dem Druck ab, mit dem ein Gasgemisch in die Eingabeeinheit eingegeben worden ist.

Das Analysegerät vermag den Druck an der ersten Messposition, bevorzugt den zeitlichen Verlauf dieses Drucks, zu ermitteln und hierfür mindestens einen Messwert, bevorzugt ein Signal, also eine Abfolge von Messwerten, des Drucksensors zu verwenden. Der Druck an der ersten Messposition stimmt - in der Regel nach Ablauf einer Einschwingphase - mit dem Druck in der Messkammer überein, und zwar in der Regel sowohl bei geöffnetem als auch bei geschlossenem Ventil. Solange das Ventil geschlossen ist, kann der Druck an der ersten Messposition vom Umgebungsdruck und vom Druck in der Eingabeeinheit abweichen

Optional vermag der Drucksensor zusätzlich den Druck an einer zweiten Messposition zu messen. Die zweite Messposition befindet sich ebenfalls in oder an der Fluidführungseinheit, aber flussaufwärts vom Filter. Bei aufgesetzter Eingabeeinheit befindet die zweite Messposition sich also zwischen der Ansaugöffnung und dem Filter. Das Analysegerät vermag den Druck, bevorzugt den zeitlichen Verlauf des Drucks, an der zweiten Messposition zu ermitteln und hierfür mindestens einen weiteren Messwert, bevorzugt das Signal, des Drucksensors zu verwenden.

Gesehen in die Strömungsrichtung einer Gasprobe aus der Eingabeeinheit in die Messkammer hinein befindet die erste Messposition sich also flussabwärts und die optionale zweite Messposition flussaufwärts vom Filter. Anders formuliert: Der Filter befindet sich zwischen den beiden Messpositionen.

Anmerkung: Die Begriffe "flussaufwärts" und "flussabwärts" beziehen sich auf die Fließrichtung einer Gasprobe aus der Eingabeeinheit in die Messkammer.

Das Analysegerät vermag dem aktuellen pneumatischen Widerstand des Filters zu ermitteln. Der pneumatische Widerstand des Filters ist der Quotient aus der Druckdifferenz (Zähler) und dem Volumenfluss durch den Filter (Nenner), wobei die Druckdifferenz die Druckdifferenz zwischen dem Druck flussaufwärts und dem Druck flussabwärts des Filters ist, also der Druckabfall am Filter. In vielen Fällen ist die Annahme gerechtfertigt, dass der pneumatische Widerstand nicht wesentlich vom Volumenfluss abhängt und daher die Druckdifferenz als proportional zum Volumenfluss angenommen werden kann.

Nachfolgend werden Ausgestaltungen beschrieben, wie das Analysegerät automatisch dem pneumatischen Widerstand des Filters zu ermitteln vermag.

Bevorzugt vermag das Analysegerät - genau gesagt: eine signalverarbeitende Auswertungseinheit des Analysegeräts, beispielsweise ein Steuergerät - einen Volumenfluss durch den Filter und damit durch die Fluidführungseinheit zu ermitteln. Dieser Volumenfluss tritt auf, wenn die Eingabeeinheit aufgesetzt und das Ventil geöffnet und die Ansaugeinheit aktiviert ist.

Unterschiedliche Realisierungsformen, wie das Analysegerät den Volumenfluss zu messen oder anderweitig zu ermitteln vermag, sind möglich. Möglich ist auch, dass die Auswertungseinheit anstelle oder zusätzlich zum Volumenfluss einen Massefluss ermittelt. Der Volumenfluss ist das Volumen pro Zeiteinheit, das durch eine Fluidführungseinheit fließt, der Massefluss die Masse pro Zeiteinheit.

In einer ersten Realisierungsform ermittelt das Analysegerät den Volumenfluss abhängig von einer Ansteuerung und / oder einer Eigenschaft der Ansaugeinheit. Diese Ansaugeinheit bewirkt durch das Ansaugen den Volumenfluss. In der Regel legt die Geometrie der Ansaugeinheit fest, welches Volumen eine angesaugte Gasprobe aufweist. Aus der Ansteuerung resultiert die Dauer eines Ansaugvorgangs. Der Quotient liefert wenigstens näherungsweise den gesuchten Volumenfluss. In einer anderen Realisierungsform umfasst das Analysegerät einen Volumenfluss-Sensor.

In einer zweiten Realisierungsform leitet das Analysegerät den Volumenfluss unter Verwendung des gemessenen Drucks an der ersten Messposition und des gemessenen Drucks an der zweiten Messposition her. Aus den Messwerten des Drucksensors leitet das Analysegerät einen zeitlichen Verlauf der Differenz zwischen den Drücken an den beiden Messpositionen her. Diese Druckdifferenz ist der Druckabfall am Filter. Aus dem zeitlichen Verlauf der Druckdifferenz leitet das Analysegerät den Volumenfluss her. Falls der pneumatische Widerstand des Filters auf eine andere Weise gemessen worden ist, leitet das Analysegerät den Volumenfluss aus der Druckdifferenz und dem pneumatischen Widerstand her.

In einer dritten Realisierungsform ist das Analysegerät dazu ausgestaltet, den Volumenfluss durch den Filter durch folgende Schritte näherungsweise zu ermitteln:
- Der Drucksensor misst wiederholt den Druck an der ersten Messposition.
- Das Analysegerät ermittelt eine Zeitspanne, wobei in dieser Zeitspanne an der ersten Messposition ein ausreichend großer Unterdruck relativ zu einem Referenzdruck auftritt. Um die Zeitspanne zu ermitteln, verwendet das Analysegerät den zeitlichen Verlauf des Drucks an der ersten Messposition.
- Der Referenzdruck ist beispielsweise der Druck, den der Drucksensor an der ersten Messposition an mindestens einem Zeitpunkt gemessen hat, an dem das Ventil geschlossen war und daher keine Gasprobe angesaugt wurde. Der Referenzdruck kann auch ein gemessener Druck in einer Umgebung des Analysegeräts sein.
- Der Unterdruck bewirkt, dass die Gasprobe angesaugt wird. Aus der ermittelten Zeitspanne leitet das Analysegerät die Zeitdauer her, die verwendet wird, um die Gasprobe anzusaugen.
- Das Analysegerät ermittelt das Volumen der angesaugten Gasprobe. Das Volumen der angesaugten Gasprobe ist mit ausreichender Genauigkeit durch die Geometrie und / oder die Konstruktion der Ansaugeinheit festgelegt, insbesondere durch das Volumen einer Kammer der Ansaugeinheit, und die Geometrie und die Konstruktion sind vorab bekannt. In der Regel hängt das Volumen der Gasprobe nicht relevant vom Volumen des Gasgemischs, das in die Eingabeeinheit eingegeben worden ist, ab
- Als Volumenfluss verwendet die Ansaugeinheit den Quotienten aus dem Volumen der Gasprobe und der ermittelten Zeitdauer.

Die dritte Realisierungsform erfordert nicht, dass der Druck an einer zweiten Messposition flussaufwärts vom Filter gemessen oder die Dauer eines Ansaugvorgangs aus der Ansteuerung der Ansaugeinheit hergeleitet wird. Außerdem erfordert die dritte Realisierungsform nicht, dass der pneumatische Widerstand des Filters bekannt ist. Vielmehr lässt sich mittels der dritten Realisierungsform der pneumatische Widerstand herleiten.

Gemäß einer vierten Realisierungsform umfasst das Analysegerät zusätzlich einen Volumenfluss-Sensor, wobei der Volumenfluss-Sensor den Volumenfluss durch den Filter hindurch misst und hierfür das Prinzip der thermischen Anemometrie oder das Prinzip der Laser-Doppler-Anemometrie verwendet. Auch die vierte Realisierungsform erfordert nicht, dass der Druck an der zweiten Messposition gemessen wird oder der pneumatische Widerstand des Filters bekannt ist. Vielmehr lässt sich auch gemäß der vierten Realisierungsform der pneumatische Widerstand herleiten.

Nachfolgend wird eine Anwendung der gerade beschriebenen Ausgestaltungen dargelegt, durch die der Volumenfluss ermittelt wird. Das Analysegerät - genau gesagt: eine signalverarbeitende Auswertungseinheit des Analysegeräts, beispielsweise ein Steuergerät - vermag den aktuellen pneumatischen Widerstand des Filters zu ermitteln. Für die nachfolgend beschriebene Anwendung wird die Annahme verwendet, dass der pneumatische Widerstand nicht vom Volumenfluss abhängt und daher der Druckabfall am Filter proportional zum Volumenfluss ist.

Weiter oben wurden Realisierungsformen beschrieben, um den Volumenfluss zu ermitteln, ohne den pneumatischen Widerstand des Filters zu kennen. Unterschiedliche Realisierungsformen sind möglich, wie das Analysegerät den Druckabfall am Filter misst.

In einer Realisierungsform misst der Drucksensor den Druck an der ersten Messposition und den Druck an der zweiten Messposition, wobei die erste Messposition flussabwärts und die zweite Messposition flussaufwärts vom Filter angeordnet ist. Die Differenz zwischen den beiden gemessenen Drücken liefert den Druckabfall.

In einer anderen Ausgestaltung verwendet das Analysegerät die oben erwähnte Zeitspanne, wobei in dieser Zeitspanne an der ersten Messposition ein Unterdruck relativ zu einem Referenzdruck auftritt. Um aus dieser ermittelten Zeitspanne den Druckabfall herzuleiten, wird bevorzugt folgende Annahme verwendet: Falls in der Fluidführungseinheit kein Filter angeordnet wäre, so würde folgender Ablauf auftreten:
- Die Ansaugeinheit saugt die Gasprobe an.
- Das Ventil wird geöffnet.
- Nach dem Öffnen des Ventils tritt eine Einschwingphase auf, in der Druckdifferenzen entlang der Fluidführungseinheit verschwinden. Nach Ablauf dieser Einschwingphase stimmt der Druck in der Messkammer mit dem Druck in der Fluidführungseinheit und dort flussaufwärts vom Filter überein. Insbesondere stimmt der Druck in der Messkammer mit dem Druck an der ersten Messposition überein.

Nach Ablauf der Einschwingphase wird ein Unterschied zwischen dem Druck an der Ansaugöffnung und dem Druck an der ersten Messposition im Wesentlichen von dem pneumatischen Widerstand des Filters festgelegt. Daher verwendet das Analysegerät bevorzugt als Druckabfall einen in einem Zeitraum gemittelten oder auch maximalen Unterschied zwischen dem Druck an der ersten Messposition und dem oben erwähnten Referenzdruck. Dieser Zeitraum liegt in der Zeitspanne, in der die Gasprobe angesaugt wird, und liegt nach der Einschwingphase.

Weiter oben wurden unterschiedliche Realisierungsformen beschrieben, wie das Analysegerät den Volumenstrom durch den Filter hindurch misst oder anderweitig ermittelt, ohne den pneumatischen Widerstand zu verwenden. Das Analysegerät ermittelt den aktuellen pneumatischen Widerstand des Filters als Quotient aus dem Druckabfall am Filter und dem Volumenfluss durch den Filter.

Dadurch, dass der Filter Partikel aus der durchströmenden Gasprobe herausfiltert, vergrößert sich in der Regel der pneumatische Widerstand des Filters Bevorzugt führt das Steuergerät die Ermittlung des aktuellen pneumatischen Widerstands wiederholt durch, beispielsweise stets dann, wenn seit der letzten Ermittlung des pneumatischen Widerstands N Gasproben angesaugt worden sind, wobei N >= 1 eine vorgegebene Anzahl ist, oder wenn die Gasproben, die seit der letzten Ermittlung durch den Filter hindurchgeflossen sind, insgesamt ein Volumen und / oder eine Masse oberhalb einer vorgegebenen oberen Schranke aufweisen.

Bevorzugt ist das Analysegerät wie folgt ausgestaltet: Wenn der pneumatische Widerstand außerhalb eines vorgegebenen Wertebereichs liegt, so generiert das Analysegerät eine entsprechende Meldung. Der Wertebereich ist mindestens durch eine obere Schranke gekennzeichnet, optional zusätzlich durch eine untere Schranke, die größer als Null ist. Falls der pneumatische Widerstand oberhalb der oberen Schranke liegt, so umfasst die Meldung den Hinweis, dass der Filter ausgetauscht werden muss. Das Analysegerät gibt diese Meldung in mindestens einer von einem Menschen wahrnehmbaren Form aus, bevorzugt auf einer Ausgabeeinheit des Analysegeräts selbst. Diese Meldung richtet sich also an einen Benutzer des Analysegeräts. Möglich ist auch, dass diese Meldung zusätzlich oder stattdessen an einen räumlich entfernten Empfänger übermittelt und auf einer Ausgabeeinheit des Empfängers ausgegeben wird.

In einer Realisierungsform vergleicht das Analysegerät dem pneumatischen Widerstand nicht nur mit der vorgegebenen oberen Schranke, sondern außerdem mit einer vorgegebenen unteren Schranke. Diese untere Schranke wird beispielsweise wie folgt vorgegeben: Wenn der Filter korrekt eingesetzt ist und das Analysegerät noch nicht benutzt worden ist, sich also noch keine Partikel auf dem Filter abgesetzt haben, so ist der pneumatische Widerstand des Filters gleich der oder größer als die untere Schranke. Wenn der gemessene pneumatische Widerstand also kleiner als die untere Schranke ist, so liegt ein Fehler vor. Mögliche Ursachen für diesen Fehler sind insbesondere die folgenden:
- In die Fluidführungseinheit ist überhaupt kein Filter eingesetzt
- Der Filter ist nicht korrekt eingesetzt, sodass ein Teil der abgesaugten Gasprobe den Filter umgehen kann.
- Der Filter ist beschädigt, weist beispielsweise einen Riss auf, sodass ein Teil der Gasprobe durch den Filter hindurchfließen kann, ohne dass der Filter Partikel herausfiltert.

Bevorzugt wird auch bei einem zu kleinen pneumatischen Widerstand eine Meldung generiert und ausgegeben.

In einer Ausgestaltung vermag das Analysegerät automatisch zu prüfen, ob ein Filter in den Grundkörper eingesetzt ist oder nicht, beispielsweise mittels eines Kontaktschalters oder indem das Analysegerät den pneumatischen Widerstand in der Fluidführungseinheit ermittelt. In einer anderen Ausgestaltung vermag das Analysegerät eine Bestätigung eines Benutzers zu erfassen, einen Filter eingesetzt zu haben. Möglich ist, dass die Ansaugeinheit nur dann aktiviert wird und nur dann eine Gasprobe ansaugt, wenn ein Filter eingesetzt ist und bevorzugt der pneumatische Widerstand im vorgegebenen Wertebereichs liegt.

Ein Filter mit einem hohen pneumatischen Widerstand kann dazu führen, dass eine Gasprobe zu langsam und / oder mit einem zu geringen Volumen in die Messkammer eingesaugt wird und dadurch eine falsche, insbesondere eine zu geringe, Konzentration der Substanz gemessen wird. Weitere unerwünschte Wirkungen sind die folgenden:
- Eine höhere kinetische Energie wird zum Ansaugen benötigt.
- Die Eingabe des Gasgemischs erfordert relativ viel Zeit.

Eine hohe benötigte kinetische Energie kann die Folge haben, dass relativ viel elektrische Energie verbraucht wird. Dies ist insbesondere dann von Nachteil, während das Analysegerät nicht mit einem stationären Spannungsversorgungsnetz verbunden ist und daher von einer eigenen Spannungsversorgungseinheit aufweist. Wie bereits dargelegt, hat es insbesondere folgende Nachteile, wenn eine Gasprobe aus einer eingegebenen Atemprobe relativ langsam angesaugt wird: Die Gasprobe stammt dann oft nicht mehr nur aus einem gewünschten Teil des Atemsystems des Probanden, also beispielsweise aus den oberen Atemwegen oder aus der Lunge, sondern auch aus einem anderen Teil.

Die Ausgestaltung, dass der pneumatische Widerstand des Filters gemessen wird, ermöglicht es, dass das Analysegerät selbst automatisch überprüft, ob der Filter weiterverwendet werden kann oder aber sich so stark mit herausgefilterten Partikeln zugesetzt hat, dass der Filter ausgetauscht werden muss. Nicht erforderlich ist es, den Filter rein zeitbasiert oder ausschließlich abhängig von der Anzahl der abgesaugten Gasproben auszutauschen, also unabhängig vom aktuellen Zustand des Filters. Möglich, aber dank der Ausgestaltung nicht zwingend erforderlich ist, dass ein Mensch den Filter visuell auf seinen aktuellen Zustand überprüft. Die gerade beschriebene Ausgestaltung, bei der der pneumatische Widerstand des Filters gemessen wird, ist insbesondere dann von Vorteil gegenüber einer visuellen Inspektion, wenn die herausgefilterten Partikel sich visuell nur wenig von einem Filterelement des Filters unterscheiden. Außerdem ist es nicht erforderlich, den Filter zu wiegen, um seinen aktuellen Zustand zu ermitteln.

Bevorzugt ist der Filter als ein elektrostatisch aufgeladenes und / oder mechanisch wirkendes Element ausgestaltet. Der Filter nimmt bei beiden Ausgestaltungen keinen chemischen oder thermischen Einfluss auf eine Gasprobe, die durch den Filter hindurchfließt, idealerweise überhaupt keinen Einfluss. Daher verfälscht der Filter auch nicht ein Messergebnis des Analysegeräts.

Erfindungsgemäß vermag der Sensor die Konzentration der Substanz in einer Gasprobe, die sich in der Messkammer befindet, zu messen. Unterschiedliche Wirkprinzipien für diesen Sensor sind möglich.

Beispielsweise ist die Substanz ein brennbares Gas, und der Sensor ist ein elektrochemischer Sensor und arbeitet nach dem Prinzip einer Brennstoffzelle. Die erzeugte elektrische Ladung ist ein Maß für den Gehalt des brennbaren Gases in der Gasprobe. Der Sensor kann auch ein photo-elektrischer Sensor sein. Elektromagnetische Strahlung durchdringt die Messkammer, und die zu detektierende Substanz schwächt in einem bestimmten Wellenlängenbereich elektromagnetische Strahlung ab. Die gemessene Abschwächung korreliert also mit dem Gehalt der Substanz. Der Sensor kann auch ein photo-akustischer Sensor sein. Elektromagnetische Strahlung verursacht einen akustischen Effekt, und die Substanz verändert diesen akustischen Effekt. Bei einem Photo-Ionisations-Detektor ionisiert die elektromagnetische Strahlung Moleküle. Möglich ist auch, dass der Sensor als sogenannter Wärmetönungs-Sensor ausgestattet ist, wobei ein Detektor eine brennbare Substanz oxidiert, die Oxidation der brennbaren Substanz Wärmeenergie freisetzt und die freigesetzte Wärmeenergie gemessen wird und ein Maß für die Konzentration der brennbaren Substanz ist.

In einer Realisierungsform umfasst das Analysegerät zwei Sensoren, die bezüglich der Gasprobe parallel oder auch in Reihe geschaltet sind. Diese beiden Sensoren können das gleiche oder unterschiedliche Messprinzipien anwenden. Die Ausgestaltung mit zwei unterschiedlich arbeitenden Sensoren schafft Redundanz und vergrößert in vielen Fällen die Zuverlässigkeit, dass die Konzentration und / oder die Menge der Substanz korrekt gemessen wird.

Die Erfindung betrifft weiterhin eine Überwachungseinheit, welche ein erfindungsgemäßes Analysegerät zu überwachen vermag. Die Überwachungseinheit umfasst den weiter oben beschriebenen Drucksensor sowie eine signalverarbeitende Auswertungseinheit. Die Auswertungseinheit kann ein Bestandteil eines Steuergeräts des Analysegeräts sein oder auch räumlich vom Analysegerät beabstandet angeordnet sein. Der Drucksensor vermag den Druck an der ersten Messposition zu messen. Die Auswertungseinheit vermag ein Signal des Drucksensors zu empfangen und den aktuellen pneumatischen Widerstand des Filters zu ermitteln und mit dem vorgegebenen Wertebereich zu vergleichen. Falls der pneumatische Widerstand außerhalb des Wertebereichs liegt, so generiert die Auswertungseinheit eine Meldung und bewirkt, dass diese in einer von einem Menschen wahrnehmbaren Form ausgegeben wird.

Im Folgenden wird die Erfindung anhand eines Ausführungsbeispiels beschrieben. Hierbei zeigt
- Figur 1: schematisch die Wirkungsweise eines elektrochemischen Sensors;
- Figur 2: in einer perspektivischen Darstellung schräg von oben eine erste Ausgestaltung des erfindungsgemäßen Analysegeräts;
- Figur 3: in einer Darstellung senkrecht von oben das Analysegerät von Figur 2;
- Figur 4: in einer Querschnittsdarstellung das Analysegerät von Figur 2, wobei die Eingabeeinheit fortgelassen ist;
- Figur 5: in einer perspektivischen Darstellung fast senkrecht von oben eine zweite Ausgestaltung des erfindungsgemäßen Analysegeräts;
- Figur 6: in einer Querschnittsdarstellung das Analysegerät von Figur 5;
- Figur 7: in einer perspektivischen Darstellung fast senkrecht von oben eine dritte Ausgestaltung des erfindungsgemäßen Analysegeräts, wobei die Eingabeeinheit fortgelassen ist;
- Figur 8: in einer weiteren Querschnittsdarstellung das Analysegerät von Figur 7, wobei die Eingabeeinheit fortgelassen ist.

Das erfindungsgemäße Analysegerät vermag ein Gasgemisch auf eine vorgegebene Substanz zu untersuchen. Im Ausführungsbeispiel ist das Gasgemisch eine Atemprobe, die ein zu untersuchender Proband ausgeatmet hat. Die Substanz ist im Ausführungsbeispiel Atemalkohol. Die Aufgabe ist, den Probanden daraufhin zu untersuchen, ob sich in seinem Blut Alkohol oberhalb einer Nachweisgrenze befindet oder nicht. Falls der Proband Alkohol zu sich genommen hat und der Alkohol im Blut noch nicht vollständig abgebaut ist, so enthält bekanntlich die abgegebene Atemprobe Atemalkohol. Die Erfindung lässt sich auch für eine andere Substanz verwenden, die sich in einer Atemprobe eines Probanden nachweisen lässt.

Der Proband gibt eine Atemprobe in eine Eingabeeinheit ein. Ein Teil der Atemprobe wird aus der Eingabeeinheit abgesaugt und fließt in eine Messkammer. Ein Sensor in oder an der Messkammer misst den Gehalt von Atemalkohol in der Gasprobe. Genauer gesagt: Der Sensor misst eine physikalische Detektionsgröße, die mit dem Gehalt (der Konzentration) von Atemalkohol in der Gasprobe, welche sich in der Messkammer befindet, korreliert und daher ein Maß für den Alkoholgehalt ist.

Der Sensor erzeugt ein Signal. Das erzeugte Signal umfasst eine Information über den gemessenen Atemalkohol-Gehalt. Beispielsweise misst der Sensor die Menge von Atemalkohol in der Gasprobe, und eine signalverarbeitende Auswertungseinheit leitet aus der Atemalkohol-Menge und dem Volumen der Messkammer die Konzentration von Atemalkohol in der Gasprobe und damit in der Atemprobe her.

Im Ausführungsbeispiel ist das Analysegerät ein Gerät, welches ein Mensch in der Hand halten und dem Probanden vor das Gesicht halten kann. Das Analysegerät umfasst eine eigene Spannungsversorgungseinheit und eine eigene Ausgabeeinheit. Der Proband gibt die Atemprobe in die Eingabeeinheit ein. Der gemessene Atemalkoholgehalt wird auf der Ausgabeeinheit in mindestens einer von einem Menschen wahrnehmbaren Form angezeigt. Optional wird zusätzlich angezeigt, ob der gemessene Atemalkohol-Gehalt oberhalb einer vorgegebenen Schranke liegt. Diese Schranke ist beispielsweise durch gesetzliche Vorschriften für Autofahrer und andere Fahrzeugführer oder Anlagenführer vorgegeben.

Aus dem Stand der Technik sind unterschiedliche Prinzipien bekannt geworden, wie ein Sensor die Konzentration einer Substanz in einem Gasgemisch zu messen vermag. Mehrere dieser Prinzipien lassen sich auch für die Erfindung anwenden. Der Sensor des Analysegeräts gemäß dem Ausführungsbeispiel ist beispielsweise ein elektro-chemischer Sensor, ein photo-optischer Sensor, ein photo-akustischer Sensor, ein Photo-Ionisierungs-Sensor oder ein Wärmetönungs-Sensor (katalytischer Sensor).

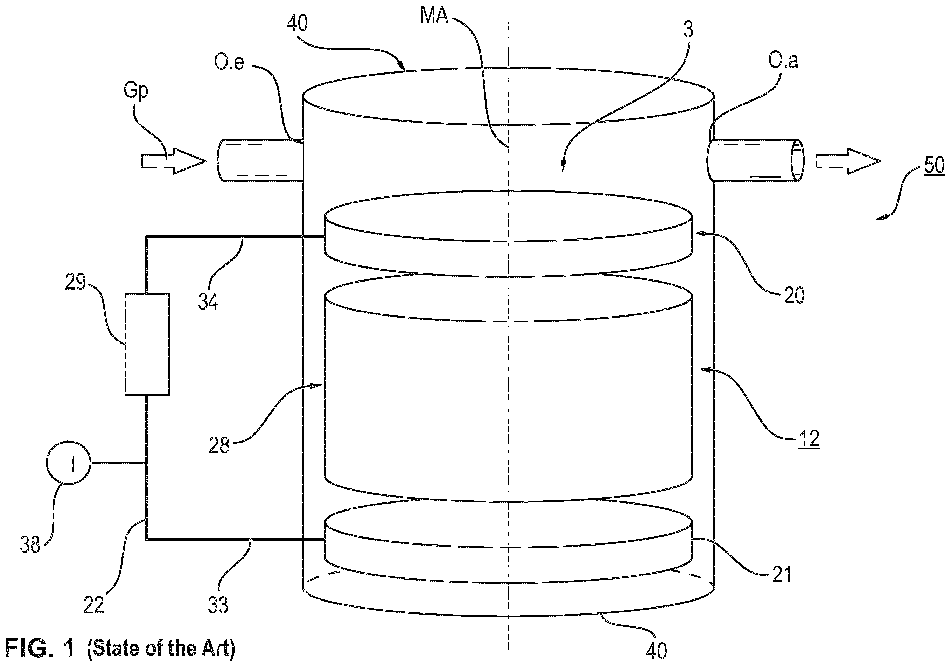

In einer Realisierungsform umfasst das Analysegerät einen elektro-chemischen Sensor. Figur 1 zeigt schematisch und beispielhaft die Wirkungsweise eines elektrochemischen Sensors 12, wobei diese Wirkungsweise aus dem Stand der Technik bekannt ist. Der Sensor 12 arbeitet nach dem Prinzip einer Brennstoffzelle mit Atemalkohol als dem Brennstoff. Atemalkohol erzeugt eine chemische Reaktion in der Messkammer. Diese chemische Reaktion löst den Schritt aus, dass elektrischer Strom fließt. Die elektrische Ladung wird gemessen und ist ein Maß für den Gehalt von Atemalkohol in der Gasprobe Gp, welche sich in einer Messkammer 3 befindet.

Die Darstellung von Figur 1 ist nicht notwendigerweise maßstabgerecht. Mit dem Bezugszeichen 50 wird eine Sensor-Anordnung bezeichnet. Die Sensor-Anordnung 50 umfasst den Sensor 12 und der Messkammer 3, die von einer Wandung 40 umgeben wird. Im Ausführungsbeispiel hat die Messkammer 3 die Form eines Zylinders, der rotationssymmetrisch zu einer Mittelachse MA ist. Selbstverständlich sind auch andere geometrische Formen möglich.

Eine Gasprobe Gp fließt durch einen Einlass O.e in die Messkammer 3 hinein und durch einen Auslass O.a wieder aus der Messkammer 3 heraus. Möglich ist auch, dass die Gasprobe Gp wieder aus dem Einlass O.e hindurch aus der Messkammer 3 herausfließt.

Der elektrochemischer Sensor 12 umfasst
- eine Messelektrode 20, die durch einen Kontaktierungsdraht 34 elektrisch kontaktiert ist,
- eine Gegenelektrode 21, die durch einen Kontaktierungsdraht 33 elektrisch kontaktiert ist,
- einen Elektrolyten 28 zwischen den beiden Elektroden 20 und 21,
- einen Verbindungsdraht 22, der die beiden Kontaktierungsdrähte 33 und 34 elektrisch miteinander verbindet,
- ein elektrischer Messwiderstand 29 im Verbindungsdraht 22 und
- eine Stromstärken-Sensor 38, der die Stärke I des Stroms, der durch den Verbindungsdraht 22 fließt, misst.

Der Elektrolyt 28 umfasst ein elektrolytisch leitendes Medium, beispielsweise mit Wasser verdünnte Schwefelsäure oder Phosphorsäure oder Perchlorsäure. In eine Realisierungsform stellt eine poröse Membrane den Elektrolyten 28 bereit. Im Elektrolyten 28 können sich Ionen bewegen. Der Elektrolyt 28 stellt eine ionisch leitfähige Verbindung zwischen der Messelektrode 20 und der Gegenelektrode 21 her, unterbindet aber den Fluss von Elektronen zwischen den beiden Elektroden 20 und 21. Die Gasprobe Gp erreicht die Messelektrode 20, aber nicht die Gegenelektrode 21. Die beiden Kontaktierungsdrähte 33 und 34 sind elektrisch leitend und aus einem Material, welches vom Elektrolyten 28 nicht chemisch angegriffen wird, beispielsweise aus Platin oder Gold. Auch die Elektroden 20 und 21 sind aus einem chemisch resistenten Material, beispielsweise ebenfalls aus Platin oder Gold.

Wie bereits dargelegt, löst die zu detektierende Substanz, hier Atemalkohol, eine chemische Reaktion aus, bei der die zu detektierende Substanz oxidiert wird - natürlich nur dann, wenn eine ausreichende Menge der Substanz in der Gasprobe Gp vorhanden ist. Als Folge der chemischen Reaktion fließt ein elektrischer Strom zwischen der Messelektrode 20 und der Gegenelektrode 21 und damit durch den Verbindungsdraht 22. Der Stromstärken-Sensor 38 misst die Stromstärke I. Eine Auswertungseinheit leitet die elektrische Ladung Q her, also die gesamte Menge des elektrischen Stroms, der durch den Verbindungsdraht 22 fließt (Prinzip der Coulometrie). In der Regel fließt der elektrische Strom so lange, bis die gesamte Menge der zu detektierenden Substanz, hier also der gesamte Atemalkohol oder eine andere oxidierbare Substanz, in der Messkammer 3 oxidiert ist. Die elektrische Ladung korreliert mit dem Gehalt an Atemalkohol in der Gasprobe Gp.

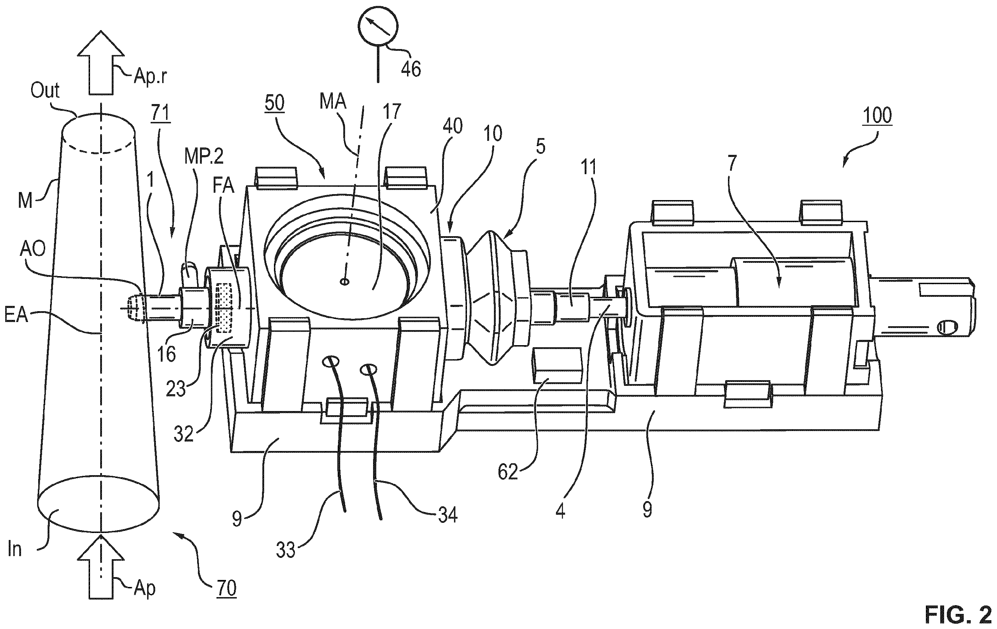

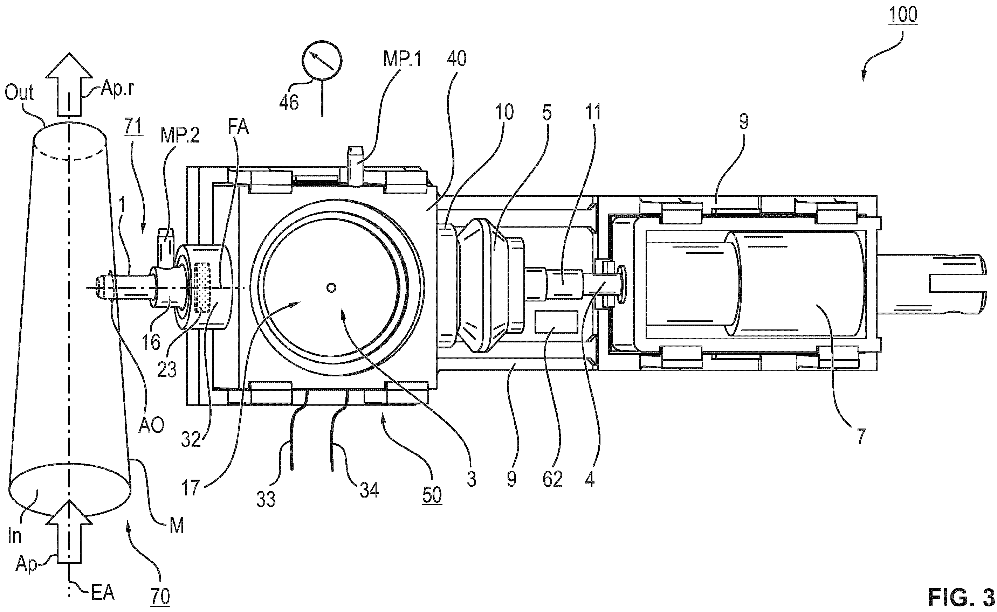

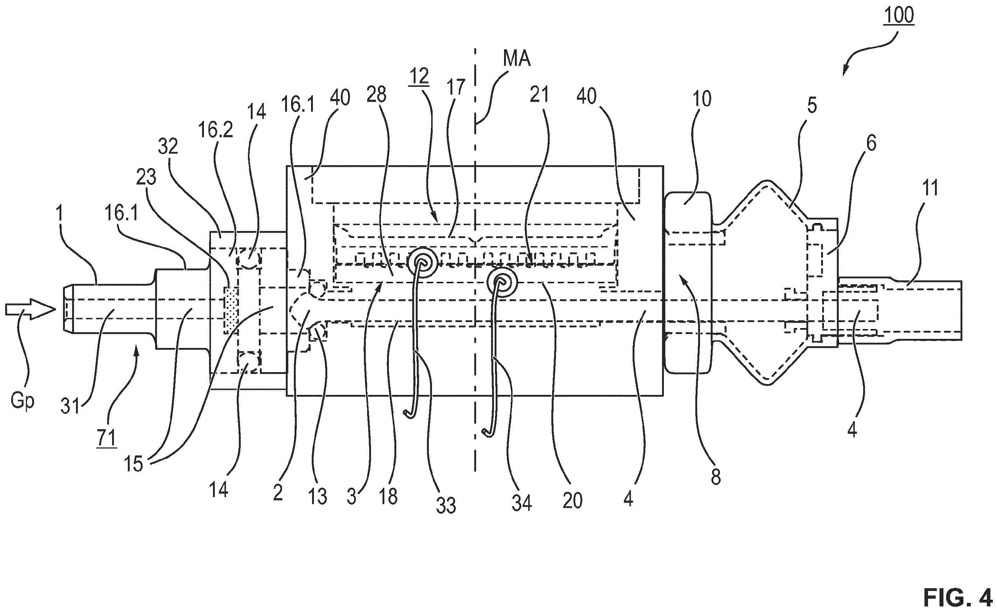

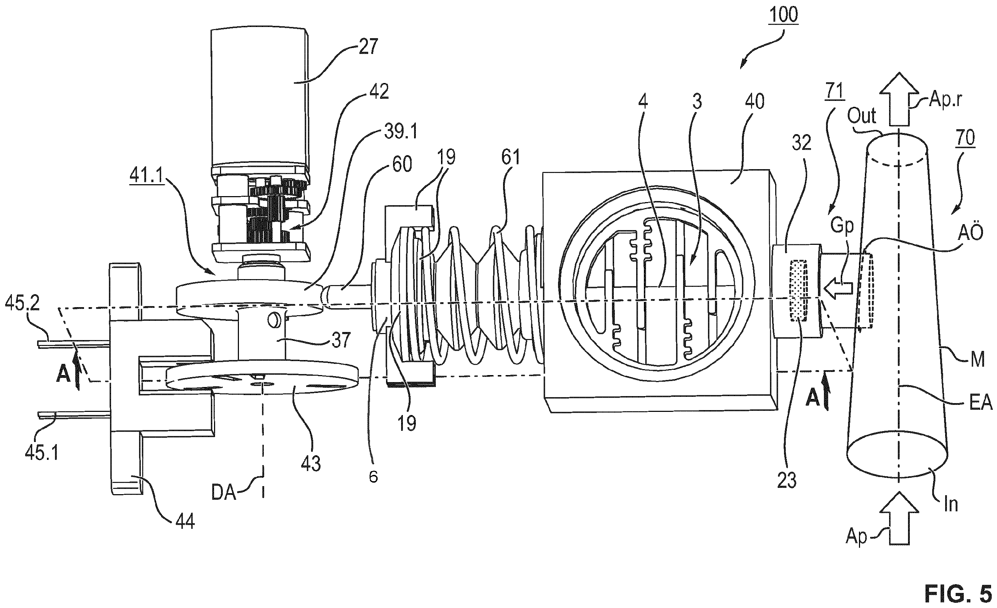

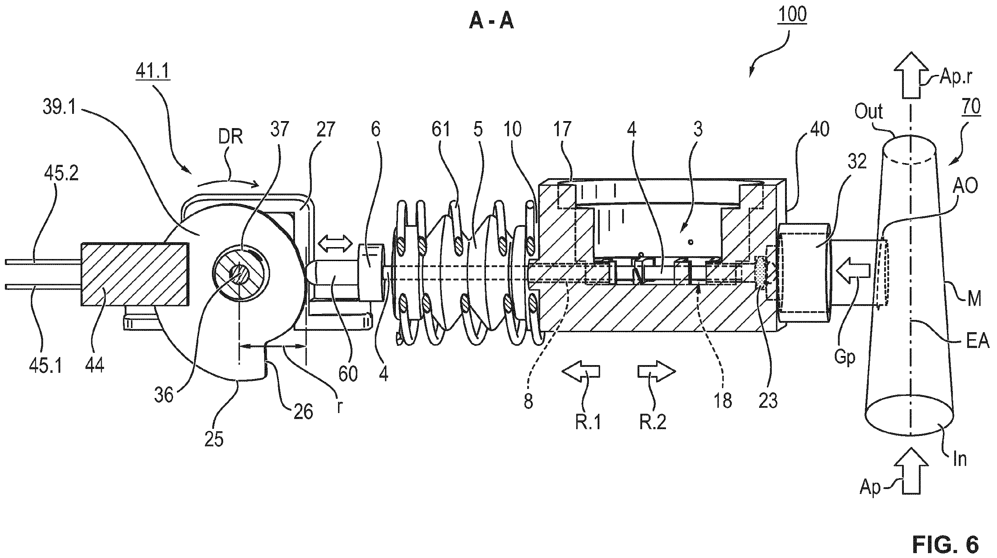

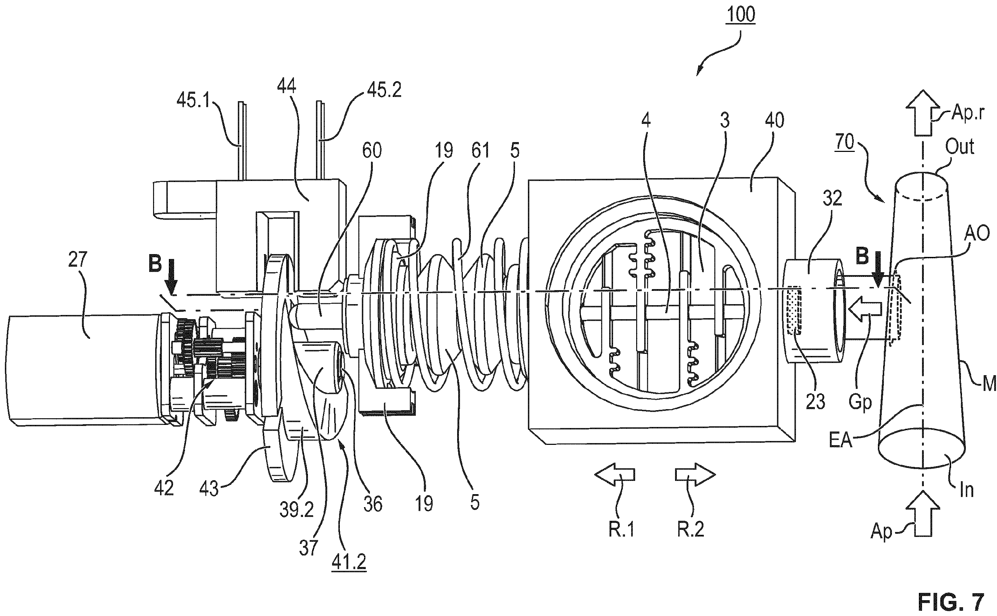

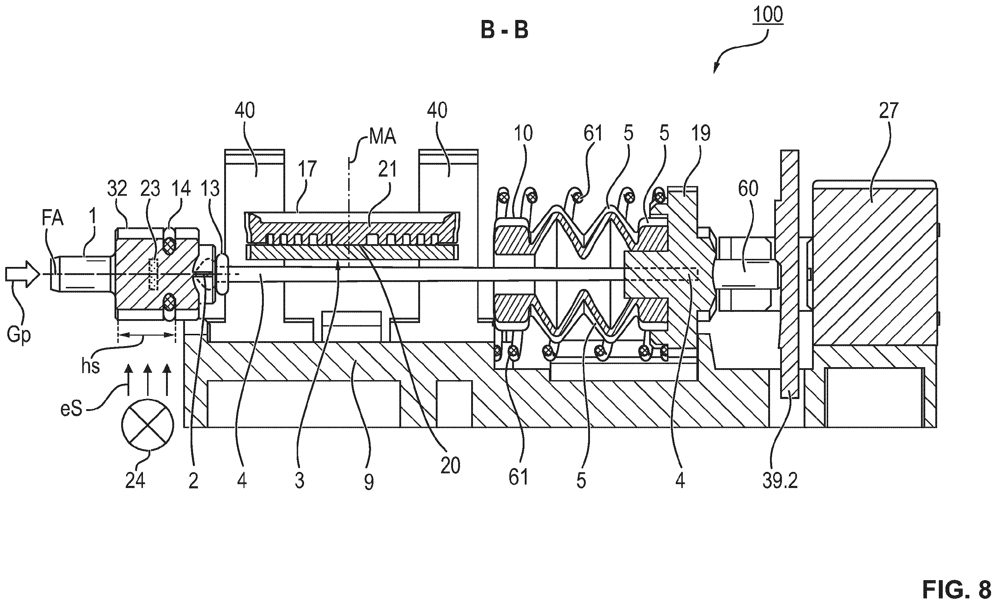

Figur 2 bis Figur 4 zeigen eine erste Ausgestaltung des erfindungsgemäßen Analysegeräts, Figur 5 und Figur 6 eine zweite Ausgestaltung und Figur 7 und Figur 8 eine dritte Ausgestaltung. Gleiche Bezugszeichen haben die gleichen Bedeutungen und auch die Bedeutungen von Figur 1. Figur 2, Figur 3, Figur 5 und Figur 7 zeigen das Analysegerät 100 in perspektivischen Darstellungen senkrecht oder schräg von oben, Figur 4 zeigt die erste Ausgestaltung in einer Querschnittsdarstellung von der Seite, und Figur 6 und Figur 8 zeigen die zweite bzw. dritte Ausgestaltung in zwei verschiedenen Querschnittsdarstellungen von der Seite.

Das Analysegerät 100 umfasst bei allen Ausgestaltungen eine röhrenförmige Eingabeeinheit 70. Die Eingabeeinheit 70 umfasst
- einen Einlass In,
- einen Auslass Out,
- eine röhrenförmige Mantelfläche M zwischen dem Einlass In und dem Auslass Out und
- eine Ansaugöffnung AO in der Mantelfläche M.

Die Eingabeeinheit 70 wird schematisch in Figur 2, Figur 3, Figur 5, Figur 6 und Figur 7 gezeigt und ist in den übrigen Figuren fortgelassen. Im Ausführungsbeispiel hat die Eingabeeinheit 70 die Form eines Trichters, der sich vom Einlass Ein zum Auslass Out hin verjüngt. Die Eingabeeinheit 70 weist eine Mittelachse EA auf. Im Ausführungsbeispiel ist die Eingabeeinheit 70 annähernd rotationssymmetrisch zur Mittelachse EA.

Wenn ein Proband eine Atemprobe Ap eingeben soll, wird das Analysegerät 100 so vor seinen Mund gehalten, dass der Einlass Ein zum Mund des Probanden zeigt. Der Proband gibt die Atemprobe Ap in die Eingabeeinheit 70 ein. Die eingegebene Atemprobe Ap fließt durch den Einlass Ein in die Eingabeeinheit 70 und parallel zur Mittelachse EA durch die Eingabeeinheit 70 hindurch zum Auslass Out. Ein Teil der Atemprobe Ap wird durch die Ansaugöffnung AO hindurch abgesaugt, was weiter unten näher beschrieben wird. Der nicht abgezweigte Rest Ap.r der Atemprobe Ap fließt durch den Auslass Out hindurch in die Umgebung. Der abgesaugte Teil der Atemprobe Ap wird später wieder in die Eingabeeinheit 70 eingespeist.

Ein Gehäuse umgibt einen Grundkörper des Analysegeräts 100. Ein Benutzer hält diesen Grundkörper in einer Hand, während der Proband die Atemprobe Ap abgibt. Vom Grundkörper ist nur ein Gestell 9 zu sehen.

Auf das Gestell 9 ist die Sensor-Anordnung 50 mit der Messkammer 3 und dem Sensor 12 aufgebracht, wobei die Sensor-Anordnung 50 so wie mit Bezug auf Figur 1 beschrieben ausgestaltet sein kann. Eine Wandung 40 umgibt die zylinderförmige Messkammer 3. Die äußere Oberfläche der Wandung 40 hat etwa die Form eines Quaders. Auf die Wandung 40 ist eine Deckplatte 17 aufgesetzt.

Eine Fluidführungseinheit 71 verbindet die Ansaugöffnung AO in der Eingabeeinheit 70 mit der Messkammer 3. Die Fluidführungseinheit 71 erstreckt sich entlang einer Längsachse FA. Im Ausführungsbeispiel steht die Längsachse FA der Fluidführungseinheit 71 senkrecht auf der Längsachse (Mittelachse) EA der verbunden Eingabeeinheit 70. Allgemein schließt die Längsachse FA mit der Mittelachse MA einen Winkel von mindestens 60° ein.

Die Fluidführungseinheit 71 umfasst
- eine hohle Spitze 1, die mit der Eingabeeinheit 70 verbunden ist,
- ein hohles Verbindungsstück 16 mit einem kleineren Teil 16.1 und einem größeren Teil 16.2 und
- ein einströmseitiges Anschlussstück 32.

Die Fluidführungseinheit 71 stellt eine Fluidverbindung zwischen der Eingabeeinheit 70 und der Messkammer 3 her. Die Fluidverbindung umfasst ein Segment 31 in der hohlen Spitze 1, ein Segment 15 im hohlen Verbindungsstück 16 und ein Segment 18 unterhalb der Messkammer 3. Das Segment 18 steht in einer Fluidverbindung mit der Messkammer 3.

In einer Ausgestaltung lassen sich auf die Spitze 1 wahlweise die Eingabeeinheit 70 mit der Ansaugöffnung AO oder ein nicht gezeigter Verschluss aufsetzen. Gemäß dieser Ausgestaltung wird der Verschluss aufgesetzt, wenn das Analysegerät 100 nicht benutzt wird und daher keine Eingabeeinheit aufgesetzt ist. Bevor eine Eingabeeinheit 70 auf die Spitze 1 aufgesetzt wird, muss der Verschluss entfernt werden. Ansonsten lässt sich in der Regel die Eingabeeinheit 70 nicht aufsetzen. Umgekehrt muss die Eingabeeinheit 70 entfernt werden, um den Verschluss aufzusetzen. Möglich, aber dank eines nachfolgend beschriebenen Filters nicht erforderlich ist es, einen solchen Verschluss zu verwenden.

Möglich ist, dass die Eingabeeinheit 70 einmal verwendet und anschließend entsorgt wird. Bei dieser Ausgestaltung ist die Eingabeeinheit 70 lösbar mit der Spitze 1 verbunden. Diese Ausgestaltung ermöglicht es, dass ein Proband die Eingabeeinheit 70 in den Mund nimmt oder sie ihm nahe vor das Gesicht gehalten wird.

Die Messkammer 3 in der Wandung 40 befindet sich zwischen der Fluidführungseinheit 71 und einer Ansaugeinheit. Die Ansaugeinheit saugt eine Gasprobe Gp aus der Eingabeeinheit 70 durch die Fluidführungseinheit 71 hindurch in die Messkammer 3. Im Ausführungsbeispiel vermag die Ansaugeinheit außerdem die Messkammer 3 auszuspülen, wobei eine "alte" Gasprobe in der Messkammer 3 durch die Fluidführungseinheit 71 hindurch und in die Eingabeeinheit 70 ausgestoßen wird. In einer Ausgestaltung wird die alte Gasprobe dann aus der Messkammer 3 ausgestoßen, wenn eine Eingabeeinheit 70 mit der Spitze 1 verbunden ist, in einer anderen Ausgestaltung dann, wenn die Spitze 1 nicht mit einer Eingabeeinheit verbunden ist.

Im Ausführungsbeispiel umfasst die Ansaugeinheit einen Balg 5 mit einem veränderbaren Volumen. Der Balg 5 ist an einem röhrenförmigen ausströmseitigen Anschlussstück 10 befestigt. Das Anschlussstück 10 ist an der Wandung 40 befestigt und stellt eine Fluidverbindung 8 zwischen der Messkammer 3 und dem Inneren des Balgs 5 her. Die Wandung 40 befindet sich zwischen den beiden Anschlussstücken 32 und 10.

Der Balg 5 hat ein veränderbares Volumen. Wenn das Volumen des Balgs 5 vergrößert wird, so entsteht im Balg 5 ein Unterdruck, dadurch wird Gas angesaugt, und eine Gasprobe Gp wird durch die Fluidführungseinheit 71 hindurch in die Messkammer 3 gesaugt. Wird umgekehrt das Volumen des Balgs 5 verkleinert, so entsteht ein Überdruck, dadurch wird Gas aus dem Balg 5 ausgestoßen, und eine Gasprobe Gp wird aus der Messkammer 3 ausgestoßen und in die Fluidführungseinheit 71 gedrückt. Dadurch wird die Messkammer 3 ausgespült. In der Regel ist das Volumen der angesaugten Gasprobe Gp etwa gleich der Differenz zwischen dem größten und dem kleinsten Volumen des Balgs 5.

Im Inneren des Balgs 5 ist eine Platte 6 angeordnet, die mit einer Hülse 11 verbunden ist, vgl. Figur 4. Die Hülse 11 ist mit einer Stange 4 verbunden. Eine lineare Bewegung der Stange 4 parallel zur Längsachse FA von der Fluidführungseinheit 71 weg vergrößert das Volumen des Balgs 5. Eine lineare Bewegung der Stange 4 in die entgegengesetzte Richtung verkleinert das Volumen des Balgs 5.

Ein Ventil vermag die Fluidführungseinheit 71 wahlweise zu verschließen oder freizugeben. Das Ventil umfasst einen Ventilkörper 2 und einen Ventilkörper-Sitz 13 in Form eines Dichtrings. Wenn das Ventil 2, 13 geschlossen ist, liegt der Ventilkörper 2 am Ventilkörper-Sitz 13 an, und zwar idealerweise fluiddicht. Wenn ein Abstand zwischen dem Ventilkörper 2 und dem Ventilkörper-Sitz 13 auftritt, gibt das Ventil 2, 13 die Fluidführungseinheit 71 frei. Nur dann, wenn das Ventil 2, 13 die Fluidführungseinheit 71 freigibt, kann eine Gasprobe Gp aus der Eingabeeinheit 70 angesaugt werden und durch die Fluidführungseinheit 71 fließen. Figur 4 zeigt das Ventil 2, 13 in einem freigebenden Zustand.

Im Ausführungsbeispiel sitzt der Ventilkörper 2 auf dem zur Eingabeeinheit 70 zeigenden Ende der Stange 4. Eine Bewegung der Stange 4 verändert nicht nur das Volumen des Balgs 5, sondern bewegt auch den Ventilkörper 2 relativ zum Ventilkörper-Sitz 13. Dank der Stange 4 wird der Vorgang, eine Gasprobe Gp durch die Fluidführungseinheit 71 hindurch anzusaugen oder die Messkammer 3 auszuspülen, mit dem Vorgang synchronisiert, das Ventil 2, 13 zu öffnen oder zu schließen. Insbesondere wird bei einer Realisierungsform ermöglicht, dass das Ventil 2, 13 nur dann geöffnet ist, wenn eine Gasprobe Gp angesaugt oder die Messkammer 3 ausgespült werden soll, und ansonsten geschlossen ist.

In einer bevorzugten Ausgestaltung wird folgende Abfolge durchgeführt, um eine neue Gasprobe Gp in die Messkammer 3 zu verbringen:
- Anfänglich ist das Ventil 2, 13 geschlossen. Der Balg 5 weist das maximale Volumen auf.
- Die Stange 4 wird auf die Eingabeeinheit 70 zubewegt. Dadurch wird das Volumen des Balgs 5 verringert, die Messkammer 3 wird ausgespült, und das Ventil 2, 13 wird geöffnet. Das zuvor in der Messkammer 3 befindliche Gas wird durch die Fluidführungseinheit 71 hindurch in die Eingabeeinheit 70 gefördert.
- Nunmehr wird die Stange 4 wieder von der Eingabeeinheit 70 wegbewegt. Das Volumen des Balgs 5 wird vergrößert, und eine Gasprobe Gp aus der Eingabeeinheit 70 wird durch die Fluidführungseinheit 71 hindurch in die Messkammer 3 gesaugt.
- Das Ansaugen der Gasprobe Gp ist abgeschlossen, und die Bewegung der Stange 4 wird beendet, wenn der Ventilkörper 2 den Ventilkörper-Sitz 13 erreicht hat und damit das Ventil 2, 13 wieder geschlossen ist.

In Figur 4 wird außerdem ein Dichtring 14 gezeigt.

Bei der ersten Ausgestaltung vermag ein Stellantrieb die Stange 4 linear in zwei antiparallele Richtungen R.1 und R.2 zu bewegen. Ein nicht gezeigtes Rückstellelement ist bestrebt, die Stange 4 von der Eingabeeinheit 70 weg zu bewegen und dadurch das Ventil 2, 13 zu verschließen und den Balg 5 in einen Zustand mit maximalem Volumen zu bewegen und in diesem zu halten. Ein Hubmagnet 7 lässt sich aktivieren, indem Strom an den Hubmagneten 7 angelegt wird, und wieder deaktivieren. Der aktivierte Hubmagnet 7 ist bestrebt, die Stange 4 auf die Eingabeeinheit 70 zu zu bewegen, und zwar gegen die Kraft des Rückstellelements, und dadurch das Volumen des Balgs 5 zu verkleinern und das Ventil 2, 13 zu öffnen. Das Aktivieren des Hubmagneten 7 bewirkt, dass die Messkammer 3 ausgespült wird. Sobald der Hubmagnet 7 wieder deaktiviert wird, bewegt das Rückstellelement die Stange 4 weg von der Eingabeeinheit 70, und die bewegte Stange 4 überführt den Balg 5 in den Zustand mit maximalem Volumen. Der Vorgang, das Ventil 2, 13 in die verschließende Endposition zu überführen und in dieser zu halten, verbraucht dank des Rückstellelements keine elektrische Energie.

Bei der ersten Ausgestaltung vermag ein Stellantrieb mit einem Hubmagneten 7 die Stange 4 linear hin und her zu bewegen. Bei der zweiten und dritten Ausgestaltung wird die Stange 4 ebenfalls linear in zwei antiparallele Richtungen R.1 und R.2 bewegt, aber nicht von einem Stellantrieb, sondern von einem Motor 27, bevorzugt von einem Elektromotor 27. Der Motor 27 dreht über ein Untersetzungsgetriebe 42 eine Ausgangswelle 36 um eine Drehachse DA, vgl. Figur 5. Im Ausführungsbeispiel ist die Drehachse DA der Ausgangswelle 36 parallel zur Mittelachse EA der Eingabeeinheit 70 angeordnet, die parallele Anordnung ist aber nicht notwendig.

Dank der nachfolgend beschriebenen Realisierungsform ist es ausreichend, dass der Motor 27 sich einschalten und ausschalten lässt und dass der eingeschaltete Motor 27 die Ausgangswelle 36 immer in dieselbe Drehrichtung DR um die Drehachse DA zu drehen vermag. Nicht erforderlich ist, einen Stellantrieb vorzusehen, der eine oszillierende Bewegung ausführt.

Eine Übertragungseinheit 41.1 (zweite Ausgestaltung) bzw. 41.2 (dritte Ausgestaltung) erzeugt aus der kontinuierlichen Drehung der Ausgangswelle 36 eine oszillierende Bewegung der Stange 4. Die Übertragungseinheit 41.1, 41.2 umfasst eine Nockenwelle 37, die drehfest mit der Ausgangswelle 36 verbunden ist. Auf der Nockenwelle 37 ist eine Nockenscheibe 39.1 (zweite Ausgestaltung) bzw. eine Nockenscheibe 39.2 (dritte Ausgestaltung) drehfest befestigt.

Die Nockenscheibe 39.1 gemäß der zweiten Ausgestaltung umfasst eine Umfangskontur, die entlang des Umfangs variiert. Mit anderen Worten: Der Abstand zwischen der Außenkontur und der Mittelachse DA der Nockenscheibe 39.1 variiert entlang ihres Umfangs. Gesehen in eine Betrachtungsrichtung parallel zur Mittelachse DA hat die Nockenscheibe 39.1 also nicht die Form eines Kreises, sondern beispielsweise einer Schnecke. Der Umfang der Nockenscheibe 39.1 umfasst ein Segment 25 mit maximalem Radius sowie eine radiale Kante 26, in der der Radius r sich schlagartig verändert, vgl. Figur 6.

Die Nockenscheibe 39.2 gemäß der dritten Ausgestaltung umfasst eine variierende Flächenkontur. Genauer gesagt: Diejenige Oberfläche der Nockenscheibe 39.2, die zur Stange 4 hin zeigt, ist gewölbt, beispielsweise stufenlos ansteigend. Diese Oberfläche hat von einer Ebene, die senkrecht auf den Drehachse DA steht, einen über der Oberfläche variierenden Abstand.

Die Stange 4 ist durch eine Halterung 19 hindurchgeführt. Auf demjenigen Ende der Stange 4, die zur Nockenscheibe 39.1, 39.2 hin zeigt, ist ein Stößel 60 befestigt. Eine Druckfeder 61 stützt sich an der Wandung 40 ab und ist bestrebt, die Stange 4 auf die Nockenscheibe 39.1, 39.2 zu zu bewegen und dadurch den Stößel 60 gegen die Umfangskontur der Nockenscheibe 39.1 (zweite Ausgestaltung) bzw. gegen die Flächenkontur der Nockenscheibe 39.2 (dritte Ausgestaltung) zu drücken und ihn in einer berührenden Position zu halten. Dadurch steht der Stößel 60 durchgehend in Kontakt mit der Umfangskontur der Nockenscheibe 39.1 bzw. der Nockenscheibe 39.2, auch wenn die Nockenscheibe 39.1, 39.2 um die Drehachse DA gedreht wird.

Außerdem ist im Ausführungsbeispiel auf der Nockenwelle 37 eine Lochscheibe 43 mit mehreren Aussparungen drehfest befestigt, vgl. Figur 5 und Figur 7. Eine Lichtschranke 44 wird mithilfe von zwei elektrischen Kontaktierungen 45.1, 45.2 mit elektrischer Energie versorgt. Eine Lichtquelle der Lichtschranke 44 emittiert einen Lichtstrahl. Je nach Rotationsposition der Lochscheibe 43 und damit der Nockenscheibe 39.1, 39.2 durchdringt der emittierte Lichtstrahl eine Aussparung in der Lochscheibe 43 und trifft auf einen Empfänger der Lichtschranke 44 auf oder wird von der Lochscheibe 43 unterbrochen. Dank der Lichtquelle lässt sich also die aktuelle Rotationsposition der Nockenscheibe 39.1, 39.2 messen. Bei der dritten Ausgestaltung bilden die Nockenscheibe 39.2 mit der variablen Flächenkontur und die Lochscheibe 43 ein einziges Bauteil, das drehfest auf der Nockenwelle 37 befestigt ist.

Dank der Lochscheibe 43 lässt sich die oszillierende Bewegung der Stange 4 relativ zuverlässig regeln. Die Regelung ermöglicht es, dass aus der Atemprobe Ap eine Gasprobe Gp abgesaugt wird, wobei die abgesaugte Gasprobe Gp ausgeatmete Luft aus mindestens einem gewünschten Bereich des Atemsystems des Probanden umfasst und idealerweise vollständig frei von ausgeatmeter Luft aus mindestens einem anderen Bereich des Atemsystems ist. Um dieses Ziel zu erreichen, wird der Motor 27 angesteuert und bewegt die Stange 4 in einer geregelten Weise. Die aktuelle Rotationsposition der Nockenscheibe 39.1, 39.2 legt die aktuelle Position der Stange 4 und damit das aktuelle Volumen des Balgs 5 fest.

Wie bereits dargelegt, verbindet eine Fluidführungseinheit 71 die Eingabeeinheit 70 mit der Messkammer 3. Im Inneren der Fluidführungseinheit 71 wird eine Fluidverbindung mit drei in Reihe geschalteten Segmenten 31, 15, 18 bereitgestellt. Ein Ventil mit einem Ventilkörper 2 und einem Ventilkörper-Sitz 13 verschließt die Fluidführungseinheit 71 oder gibt sie frei, je nachdem ob der Ventilkörper 2 am Ventilkörper-Sitz 13 anliegt oder ein Abstand auftritt. Gewünscht wird, dass das Ventil 2, 13 tatsächlich die Fluidverbindung 31, 15, 18 fluiddicht verschließt und dadurch die Messkammer 3 von der Umgebung trennt, wenn keine Gasprobe Gp abgesaugt werden soll und auch nicht die Messkammer 3 ausgespült werden soll. Dadurch wird verhindert, dass eine Substanz im Sensor verdunstet, was insbesondere bei einem elektrochemischen Sensor der Fall sein kann, oder umgekehrt ein unerwünschter Umgebungseinfluss auf einen Sensor auftreten kann, insbesondere Partikel eindringen.

Jedoch kann das unerwünschte Ereignis auftreten, das Partikel zwischen den Ventilkörper 2 und den Ventilkörper-Sitz 13 gelangen und dadurch das Ventil 2, 13 auch dann nicht vollständig fluiddicht schließt, wenn der Ventilkörper 2 am Ventilkörper-Sitz 13 anliegt. Nachfolgend wird beschrieben, wie erfindungsgemäß die Gefahr reduziert wird, dass dieses unerwünschte Ereignis auftritt.

Das Analysegerät 100 umfasst zusätzlich einen Filter 23 im Inneren der Fluidführungseinheit 71 und dort an einer Position flussaufwärts vom Ventil 2, 13, also bei aufgesetzter Eingabeeinheit 70 zwischen der Eingabeeinheit 70 und dem Ventil 2, 13. Die Gasprobe Gp, die aus der Eingabeeinheit 70 abgesaugt wird, fließt zunächst durch den Filter 23 hindurch und erreicht dann das Ventil 2, 13. Der Filter 23 filtert aus der durchfließenden Gasprobe Gp alle Partikel heraus, die größer als eine vorgegebene obere Schranke sind oder eine andere vorgegebene Eigenschaft aufweisen. Diese obere Schranke ist durch die Konstruktion des Filters 23 vorgegeben und beträgt beispielsweise 2 µm.

Im Ausführungsbeispiel befindet der Filter 23 sich im Inneren des Verbindungsstücks 16 und auch innerhalb des Anschlussstücks 32. Andere Positionen sind ebenfalls möglich. Bevorzugt ist der Filter 23 so weit weg von der Eingabeeinheit 70 angeordnet, dass die Gefahr relativ gering ist, dass der Filter 23 durch einen Einfluss von außen beschädigt wird. Andererseits ist der Filter 23 so weit entfernt von der Messkammer 3 positioniert, dass bei jeder möglichen Position der Stange 4 ein Abstand zwischen den Ventilkörper 2 und dem Filter 23 auftritt.

Typischerweise umfasst der Filter 23 ein Filterelement und eine Halterung, die das Filterelement rundum umgibt und hält. Die Halterung lässt sich in eine korrespondierende Aufnahme im Grundkörper des Analysegeräts 100 einführen und wieder herausnehmen. Die Gasprobe Gp fließt durch das Filterelement hindurch. Bevorzugt ist das Filterelement elektrostatisch aufgeladen und umfasst ein Faservlies, besonders bevorzugt ein Melt-Blown-Faservlies. Diese Ausführungsform führt zu einem Filter mit einem relativ geringen pneumatischen Widerstand und damit auch zu einem relativ geringen Druckabfall am Filter 23. Bevorzugt weist das Faservlies oder ein sonstiges Filterelement des Filters 23 eine hydrophobe Beschichtung auf oder ist aus einem hydrophoben Werkstoff gefertigt. Dadurch wird die Zuverlässigkeit erhöht, dass Feuchtigkeit in der Gasprobe Gp am Filterelement abperlt und nicht auf dem Filterelement kondensiert, das Filterelement befeuchtet oder gar den Filter 23 passiert.

Bevorzugt ist der Filter 23 in einen Schlitz im Gehäuse eingesetzt und lässt sich durch einen Zugriff von außen austauschen.

Die Gasprobe Gp stammt im Ausführungsbeispiel von einer Atemprobe Ap, die ein Proband abgibt, und weist daher eine relativ große Luftfeuchtigkeit auf. Weiter oben wurde eine Ausgestaltung beschrieben, bei der das Filterelement des Filters 23 aus einem hydrophoben Werkstoff gefertigt ist oder wenigstens eine hydrophobe Beschichtung aufweist. Diese Realisierungsform reduziert verglichen mit anderen möglichen Realisierungsformen die Gefahr, dass Flüssigkeitstropfen in der Gasprobe Gp das Filterelement beschädigen.

Eine andere oder zusätzliche mögliche Maßnahme ist die folgende: Der Filter 23 wird erwärmt, bevorzugt berührungslos erwärmt. Dank der Erwärmung bleibt die Temperatur in einem Segment hS der Fluidführungseinheit 71, in welchem sich der Filter 23 befindet, oberhalb des Taupunkts von Flüssigkeit in der Atemluft. Dadurch wird in vielen Fällen zuverlässig verhindert, dass Feuchtigkeit auf dem Filter 23 kondensiert.

Unterschiedliche Realisierungsformen einer geeigneten Heizung sind möglich. Im Ausführungsbeispiel befindet die verwendete Heizung sich außerhalb der Fluidführungseinheit 71, bevorzugt auch außerhalb des Anschlussstücks 32, und erwärmt berührungslos ein Segment hS der Fluidführungseinheit 71, vgl. Figur 8. Die schematisch gezeigte und elektrisch betriebene Heizung 24 umfasst eine Lichtquelle, beispielsweise mindestens eine LED, die wärmende elektromagnetische Strahlung eS auf den Filter 23 zu emittiert. Beispielsweise erwärmt die elektromagnetische Strahlung eS das Anschlussstück 32, und die Erwärmung des Anschlussstücks 32 wird auf dem Filter 23 übertragen. Die gezeigte Position der Lichtquelle 24 ist nur beispielhaft zu verstehen. Anstelle einer Heizung außerhalb der Fluidführungseinheit 71 lässt sich auch ein Heizwiderstand verwenden, der in das Anschlussstück 32 eingesetzt ist, insbesondere eine Heizwendel.

Im gezeigten Ausführungsbeispiel misst ein schematisch gezeigter Drucksensor 46 wiederholt den Druck an einer ersten Messposition MP.1 und optional den Druck an einer zweiten Messposition MP.2. Optional umfasst der Drucksensor 46 zwei Messkanäle für die beiden Messpositionen MP.1 und MP.2. Die erste Messposition MP.1 befindet sich flussabwärts vom Filter 23, beispielsweise in der Fluidführungseinheit 71 oder in oder an der Messkammer 3 oder zwischen der Messkammer 3 und der Ansaugeinheit 5, 6, 7, 27. Die optionale zweite Messposition MP.2 befindet sich in der Fluidführungseinheit 71 und flussaufwärts vom Filter 23, also zwischen dem Filter 23 und der Eingabeeinheit 70.

Ein signalverarbeitendes Steuergerät 62 empfängt ein Signal des Drucksensors 46 und leitet aus dem Signal den zeitlichen Verlauf des Drucks an der ersten Messposition MP.1 und optional den zeitlichen Verlauf des Drucks an der zweiten Messposition MP.2 her. Falls der Druck an beiden Messposition MP.1 und MP.2 gemessen wird, so leitet das Steuergerät 62 zusätzlich den zeitlichen Verlauf der Differenz zwischen den beiden Drücken her.

In einer Ausgestaltung leitet das Steuergerät 62 aus dem zeitlichen Verlauf der Druckdifferenz den Volumenfluss von der Ansaugöffnung AO durch die Fluidführungseinheit 71 und damit durch den Filter 23 hindurch in die Messkammer 3 her. In der Regel tritt ein nennenswerter Volumenfluss nur dann auf, wenn das Ventil 2, 13 geöffnet ist. Der Volumenfluss wird durch die Ansaugeinheit 5, 6, 7, 27 bewirkt.

In einer Anwendung leitet das Steuergerät 62 aus dem gemessenen oder anderweitig ermittelten Volumenfluss das Volumen der Gasprobe Gp in der Messkammer 3 her. Bekanntlich ist das Volumen das Integral über der Zeit und über den Volumenfluss. Die Zeitspanne, über die integriert wird, ist bevorzugt gleich der Zeitspanne, in der die Ansaugeinheit 5, 6, 7, 27 die Gasprobe Gp angesaugt und daher der der Druck an der zweiten Messposition MP.2 kleiner ist als der Druck an der ersten Messposition MP.1.

Nachfolgend wird eine weitere Anwendung des Drucksensors 46 beschrieben.

Wenn ein Gasgemisch durch einen mechanischen Filter 23 durchfließt, tritt in der Regel am Filter 23 ein Druckabfall auf. Dieser Druckabfall, also die Differenz zwischen dem Druck flussaufwärts und dem Druck flussabwärts vom Filter 23, lässt sie mit ausreichender Näherung als proportional zum Volumenfluss des durchfließenden Gasgemischs auffassen. Dieser Quotient wird als pneumatischer Widerstand des Filters 23 bezeichnet. In der Regel ist die Annahme gerechtfertigt, dass der pneumatische Widerstand nicht nennenswert vom Volumenfluss abhängt. Wenn der Volumenfluss durch den Filter 23 sowie der Druckabfall am Filter 23 bekannt sind, lässt sich der aktuelle pneumatische Widerstand des Filters 23 herleiten.

Zwangsläufig setzen sich Partikel, welche der Filter 23 aus den durchfließenden Gasproben Gp herausfiltert, auf einer Oberfläche des Filters 23 fest. Dadurch vergrößert sich der pneumatische Widerstand des Filters 23. Wenn der gemessene aktuelle pneumatische Widerstand eine obere Schranke erreicht, so sollte der Filter 23 ausgetauscht werden. Nachfolgend wird beschrieben, wie das Steuergerät 62 den pneumatischen Widerstand des Filters 23 ermittelt.

Wenn ein neuer Filter 23 eingesetzt wird, so hat er einen anfänglichen pneumatischen Widerstand. Dieser ist in der Regel durch die Konstruktion des Filters vorgegeben. Falls der gemessene pneumatische Widerstand des Filters 23 kleiner als der anfängliche pneumatische Widerstand oder sogar gleich null ist, so ist dies ein Indiz dafür, dass kein Filter eingesetztes oder der Filter 23 falsch eingesetzt oder defekt ist.

Das Steuergerät 62 misst oder ermittelt den Druckabfall am Filter 23 und den Volumenfluss durch den Filter 23 hindurch. Der Druckabfall und der Volumenfluss treten in einer Zeitspanne auf, in der das Ventil 2, 13 geöffnet ist und in der die Ansaugeinheit 5, 6, 7, 27 die Gasprobe Gp ansaugt.

Eine Ausgestaltung, um den Druckabfall und den Volumenfluss zu messen, wurde weiter oben beschrieben. Diese Ausgestaltung setzt voraus, dass wiederholt sowohl der Druck an der ersten Messposition MP.1 als auch der Druck an der zweiten Messposition MP.2 gemessen werden. Die nachfolgend beschriebene Ausgestaltung erfordert nicht, dass der Druck an der zweiten Messposition MP.2 gemessen wird.

Der pneumatische Widerstand der Fluidführungseinheit 71 ist klein im Vergleich zum pneumatischen Widerstand des Filters 23. Falls in der Fluidführungseinheit 71 kein Filter 23 vorhanden wäre, so tritt daher in der Regel folgender Ablauf auf:
- Der Antrieb 7, 27 wird aktiviert.
- Das Volumen des Balgs 5 wird vergrößert. Zugleich wird das Ventil 2, 13 geöffnet.
- Eine Gasprobe Gp wird in die Messkammer 3 gesaugt
- Nach einer in der Regel sehr kurzen Einschwingphase gleicht der Druck in der Messkammer 3 dem Druck in der Fluidführungseinheit 71 und dort flussaufwärts vom Filter 23. Anmerkung: Bekanntlich pflanzt Druck sich annähernd mit Schallgeschwindigkeit fort.

Erfindungsgemäß ist aber in der Fluidführungseinheit 71 ein Filter 23 vorhanden. Spätestens nach dem Ende der Einschwingphase wird daher ein Druckabfall in der Fluidführungseinheit 71 im Wesentlichen vom Filter 23 verursacht.

In einer Ausgestaltung ermittelt das Steuergerät 62 einen Referenzdruck an der ersten Messposition bei geschlossenem Ventil 2, 13 und bei deaktiviertem Antrieb 7, 27, beispielsweise abhängig von einem Messwert des Drucksensors 46. Wie oben dargelegt, ermittelt das Steuergerät 62 außerdem den zeitlichen Verlauf des Drucks an der ersten Messposition MP.1, während das Ventil 2, 13 geöffnet ist und die Gasprobe Gp angesaugt wird. Aus dem Referenzdruck und dem zeitlichen Verlauf des Drucks leitet das Steuergerät 62 folgende Informationen her:
- einen durchschnittlichen Druckabfall am Filter 23, während die Gasprobe Gp angesaugt wird und die Einschwingphase verstrichen ist, und
- die Zeitspanne und damit die Zeitdauer, die verwendet wird, um die Gasprobe Gp anzusaugen.

An der ersten Messposition MP.1 tritt ein Unterdruck relativ zum Referenzdruck in der Regel nur in der Zeitspanne auf, in der die Gasprobe Gp angesaugt wird.

Außerdem ermittelt das Steuergerät 62 das Volumen der angesaugten Gasprobe Gp. Die Gasprobe Gp wird im Ausführungsbeispiel dadurch angesaugt, dass das Volumen des Balgs 5 vergrößert wird. In der Regel ist das Volumen der Gasprobe Gp daher - nach einer Einschwingphase - gleich der Differenz zwischen dem maximalen Volumen und dem minimalen Volumen des Balgs 5. Diese Volumendifferenz ist aus der Geometrie und der Konstruktion der Ansaugeinheit 5, 6, 7, 27 bekannt und wird vorgegeben.

Als Volumenfluss durch den Filter 23 verwendet das Steuergerät 62 den Quotienten aus dem Volumen der Gasprobe Gp und der Zeitdauer, die zum Ansaugen der Gasprobe Gp verwendet wird.

Das Steuergerät 62 ermittelt den aktuellen pneumatischen Widerstand des Filters 23 als Quotient aus dem gemessenen oder ermittelten Druckabfall und dem gemessenen oder ermittelten Volumenfluss. Bevorzugt ermittelt das Steuergerät 62 wiederholt den jeweils aktuellen pneumatischen Widerstand. Beispielsweise ermittelt das Steuergerät 62 erneut den aktuellen pneumatischen Widerstand, wenn das Analysegerät 100 seit der letzten Ermittlung N Gasproben angesaugt hat, wobei N >= 1 eine vorgegebene Anzahl ist, oder wenn seit der letzten Ermittlung das aufsummierte Volumen der Gasproben insgesamt größer ist als eine vorgegebene obere Schranke.

Bevorzugt generiert das Analysegerät 100 eine Nachricht in mindestens einer von einem Menschen wahrnehmbaren Form, wenn der pneumatische Widerstand des Filters 23 eine vorgegebene obere Schranke erreicht hat. Diese Nachricht enthält eine Information darüber, dass der Filter 23 ausgetauscht werden muss. Das Analysegerät 100 bewirkt, dass diese Nachricht in mindestens einer von einem Menschen wahrnehmbaren Form ausgegeben wird, wenn der gemessene pneumatische Widerstand des Filters 23 diese Schranke erreicht hat. Diese Nachricht informiert einen Benutzer, dass der Filter 23 nunmehr ausgetauscht werden soll.

In einer Ausgestaltung vermag das Analysegerät 100 festzustellen, ob ein Filter 23 eingesetzt ist oder nicht. Bei nicht eingesetztem Filter ist der pneumatische Widerstand in der Fluidführungseinheit 71 signifikant geringer, insbesondere kleiner als der oben erwähnte anfängliche pneumatische Widerstand. Oder das Analysegerät 100 umfasst einen Kontaktschalter, der von einem eingesetzten Filter 23 betätigt wird. Oder das Analysegerät erfasst eine Bestätigung eines Benutzers, einen Filter 23 eingesetzt zu haben. In einer Ausgestaltung verhindert das Steuergerät 62, dass die Ansaugeinheit 5, 6 aktiviert wird und eine Gasprobe ansaugt, wenn kein Filter 23 eingesetzt ist. Außerdem generiert das Analysegerät 100 dann bevorzugt eine entsprechende Meldung.

### Bezugszeichenliste

| | |
|---|---|
| 1 | hohle Spitze der Fluidführungseinheit 71, lösbar mit der Eingabeeinheit 70 verbunden |
| 2 | Ventilkörper, auf der Stange 4 montiert, gehört zum Ventil in der Fluidführungseinheit 71 |
| 3 | zylinderförmige Messkammer, nimmt die abgesaugte Gasprobe Gp auf, von der Wandung 40 und der Deckplatte 17 umgeben, weist die Mittelachse MA auf |
| 4 | Stange, wird vom Hubmagneten 7 oder vom Motor 27 in die beiden Richtungen R.1 oder R.2 bewegt, vergrößert das Volumen des Balgs 5 und bewegt den Ventilkörper 2 relativ zum Ventilkörper-Sitz 13 |
| 5 | Balg, gehört zur Ansaugeinheit |
| 6 | Platte im Balg 5, fest mit der Hülse 11 und dadurch mit der Stange 4 verbunden |
| 7 | Hubmagnet, lässt sich aktivieren und deaktivieren, bewegt nach einer Aktivierung die Stange 4 relativ zur Fluidführungseinheit 71 und vergrößert dadurch das Volumen des Balgs 5 |
| 8 | Fluidverbindung zwischen der Messkammer 3 und dem Inneren des Balgs 5 |
| 9 | Gestell des Grundkörpers des Analysegeräts 100 |
| 10 | röhrenförmiges ausströmseitiges Anschlussstück, an der Wandung 40 befestigt |
| 11 | Hülse, mit der Stange 4 verbunden |
| 12 | elektro-chemischer Sensor, umfasst die Elektroden 20 und 21 und den Elektrolyten 28 |
| 13 | Ventilkörper-Sitz 13 in Form eines Dichtrings am Verbindungsstück 16, gehört zum Ventil in der Fluidführungseinheit 71 |
| 14 | Dichtring |
| 15 | Segment im Verbindungsstück 16, das zur Fluidführungseinheit 71 zwischen der Eingabeeinheit 70 und der Messkammer 3 gehört |
| 16 | röhrenförmiges Verbindungsstück der Fluidführungseinheit 71, verbindet die Spitze 1 mit dem Anschlussstück 32, umfasst die Teile 16.1 und 16.2 |
| 16.1 | kleinerer Teil des Verbindungsstücks 16 |
| 16.2 | größerer Teil des Verbindungsstücks 16 |
| 17 | Deckplatte auf der Wandung 40 |
| 18 | Segment unterhalb der Messkammer 3, das zur Fluidverbindung zwischen der Eingabeeinheit 70 und der Messkammer 3 gehört |
| 19 | Halterung, durch die hindurch die Stange 4 geführt ist |
| 20 | Messelektrode des Sensors 12 |
| 21 | Gegenelektrode des Sensors 12 |
| 22 | Verbindungsdraht zwischen den Kontaktierungsdrähten 33 und 34 |
| 23 | elektrostatisch aufgeladener und / oder mechanisch wirkender Filter im Segment 15 der Fluidverbindung zwischen der Umgebung und der Messkammer 3 |
| 24 | Heizung für den Filter 23, umfasst eine Strahlungsquelle in Form einer LED-Lampe, emittiert elektromagnetische Strahlung eS |
| 25 | Segment mit maximalem Radius in der Umfangskontur der Nockenscheibe 39.1 |
| 26 | radiale Kante in der Umfangskontur der Nockenscheibe 39.1 |
| 27 | Motor, dreht die Ausgangswelle 36 in die Drehrichtung DR |
| 28 | ionisch leitfähiger Elektrolyt zwischen den Elektroden 20 und 21 |
| 29 | Messwiderstand im Verbindungsdraht 22 |
| 31 | Segment in der Spitze 1, gehört zur Fluidverbindung zwischen der Eingabeeinheit 70 und der Messkammer 3 |
| 32 | einströmseitiges Anschlussstück der Fluidführungseinheit 71, an der Wandung 40 angeordnet |
| 33 | Kontaktierungsdraht für die Gegenelektrode 21 |
| 34 | Kontaktierungsdraht für die Messelektrode 20 |
| 36 | Ausgangswelle, vom Motor 27 in die Drehrichtung DR gedreht, dreht die Nockenscheibe 39.1, 39.2 |
| 37 | Nockenwelle |
| 38 | Stromstärken-Sensor, misst die Stärke des durch den Verbindungsdraht 22 fließenden Stroms |
| 39.1 | Nockenscheibe mit exzentrischer Außenkontur, drehfest auf der Nockenwelle 37 befestigt |
| 39.2 | Nockenscheibe mit exzentrischer Flächenkontur, drehfest auf der Nockenwelle 37 befestigt |
| 40 | Wandung der Messkammer 3 |
| 41.1 | Übertragungseinheit gemäß der zweiten Ausgestaltung, bei der die Nockenscheibe 39.1 eine exzentrische Außenkontur aufweist |
| 41.2 | Übertragungseinheit gemäß der dritten Ausgestaltung, bei der die Nockenscheibe 39.2 eine exzentrische Flächenkontur aufweist |
| 42 | Untersetzungsgetriebe zwischen dem Motor 27 und der Ausgangswelle 36 |
| 43 | Lochscheibe, drehfest auf der Nockenwelle 37 befestigt |
| 44 | Lichtschranke mit einer Lichtquelle und einem Empfänger, umfasst elektrischen Kontaktierungen 45.1 und 45.2 |
| 45.1, 45.2 | elektrische Kontaktierung für die Lichtschranke 44 |
| 46 | Drucksensor, misst den Druck an der ersten Messposition MP.1 und optional an der zweiten Messposition MP.2 |
| 50 | Sensor-Anordnung mit der Messkammer 3 und dem elektrochemischen Sensor 12 |
| 60 | Stößel am freien Ende der Stange 4 |
| 61 | Druckfeder, ist bestrebt, die Stange 4 gegen die Nockenscheibe 39.1, 39.2 zu drücken |
| 70 | röhrenförmige Eingabeeinheit, weist den Einlass In, den Auslass Out, die Mantelfläche M und die Mittelachse EA auf |
| 62 | Steuergerät |
| 71 | Fluidführungseinheit, stellt eine Fluidverbindung zwischen der Ansaugöffnung AO in der Eingabeeinheit 70 und der Messkammer 3 her, umfasst die Spitze 1, das Verbindungsstück 16 und das Anschlussstück 32, weist die Längsachse FA auf |
| 100 | Analysegerät, umfasst die Eingabeeinheit 70, die Fluidführungseinheit 71, die Messkammer 3 in der Wandung 40, den Sensor 12, die Ansaugeinheit 5, 6, 7, 27 und das Gehäuse mit dem Gestell 9 |
| AO | Ansaugöffnung in der Mantelfläche M der Eingabeeinheit 70 |
| Ap | Atemprobe, wird von einem Probanden durch den Einlass Ein hindurch in die Eingabeeinheit 70 eingegeben |
| Ap.r | Rest der Atemprobe Ap, der nicht abgesaugt wird und durch den Auslass Out hindurch aus der Eingabeeinheit 70 hinaus fließt |
| DA | Drehachse der Ausgangswelle 36 |
| DR | Drehrichtung, in die der Motor 27 die Ausgangswelle 36 und damit die Nockenscheibe 39.1, 39.2 dreht |
| EA | Mittelachse und Längsachse der Eingabeeinheit 70, steht senkrecht auf der Längsachse FA der Fluidführungseinheit 71 |
| eS | elektromagnetische Strahlung, von der Lichtquelle 24 emittiert, erhitzt das Segment hS |
| FA | Längsachse der Fluidführungseinheit 71, steht senkrecht auf der Mittelachse EA der Eingabeeinheit 70 |
| Gp | Gasprobe, die aus der Eingabeeinheit 70 abgesaugt (abgezweigt) wird und in die Messkammer 3 fließt, ist ein Teil der Atemprobe Ap |
| hS | Segment der Fluidführungseinheit 71, welches von der emittierten elektromagnetischen Strahlung eS erhitzt wird |
| I | Stromstärke |
| In | Einlass der Eingabeeinheit 70 |
| M | röhrenförmige Mantelfläche der Eingabeeinheit 70, weist die Ansaugöffnung AO auf |
| MA | Mittelachse der zylinderförmigen Messkammer 3 |
| MP.1 | erste Messposition: Messposition, an der der Druck flussabwärts vom Filter 23 gemessen wird, befindet sich in der Fluidführungseinheit 71 oder in oder an der Messkammer 3 oder zwischen der Messkammer 3 und der Ansaugeinheit 5, 6, 7, 27 |
| MP.2 | zweite Messposition: Messposition, an der der Druck in der Fluidführungseinheit 71 und flussaufwärts vom Filter 23 gemessen wird |
| Out | Auslass der Eingabeeinheit 70 |
| O.a | Auslass aus der Messkammer 3 |
| O.e | Einlass in die Messkammer 3 |
| R.1, R.2 | antiparallele Richtungen, in welche die Stange 4 bewegt wird |

## Patentansprüche

1. Analysegerät (100) zum Analysieren eines Gasgemischs (Ap) auf eine vorgegebene Substanz,
wobei das Analysegerät (100)
- einen Grundkörper (9),
- eine Sensor-Anordnung (50) im Inneren des Grundkörpers (9),
- eine Ansaugeinheit (5, 6, 7, 27),
- eine Fluidführungseinheit (71),
- eine röhrenförmige Eingabeeinheit (70),
- ein Ventil (2, 13) und
- einen Filter (23)
umfasst,
wobei die Sensor-Anordnung (50) eine Messkammer (3) und einen Sensor (12) umfasst,
wobei die Eingabeeinheit (70)
- einen Einlass (In), einen Auslass (Out) und eine Ansaugöffnung (AO) zwischen dem Einlass (In) und dem Auslass (Out) umfasst,
- mit dem Grundkörper (9) verbunden oder verbindbar ist, bevorzugt lösbar verbunden oder verbindbar, und
- dazu ausgestaltet ist, dass ein zu analysierendes Gasgemisch (Ap) durch den Einlass (In) in die Eingabeeinheit (70) eingegeben wird und durch die Eingabeeinheit (70) hindurch in Richtung des Auslasses (Out) fließt,
wobei dann, wenn die Eingabeeinheit (70) mit dem Grundkörper (9) verbunden ist, die Fluidführungseinheit (71) die Ansaugöffnung (AO) mit der Messkammer (3) verbindet,
wobei die Ansaugeinheit (5, 6, 7, 27) dazu ausgestaltet ist,
- aus einem Gasgemisch (Ap), das durch die Eingabeeinheit (70) hindurchfließt, eine Gasprobe (Gp) aus der Eingabeeinheit (70) durch die Ansaugöffnung (AO) hindurch anzusaugen und
- die angesaugte Gasprobe (Gp) durch die Fluidführungseinheit (71) hindurch in die Messkammer (3) zu fördern,
wobei der Sensor (12) dazu ausgestaltet ist, die Konzentration der Substanz in einer Gasprobe (Gp), die sich in der Messkammer (3) befindet, zu messen,
wobei das Ventil (2, 13)
- zwischen einer verschließenden Endposition und einer freigebenden Endposition hin und her bewegbar ist,
- in der verschließenden Endposition die Fluidführungseinheit (71) verschließt und
- in der freigebenden Endposition die Fluidführungseinheit (71) freigibt und wobei der Filter (23)
- dazu ausgestaltet ist, aus einer Gasprobe (Gp), welches durch den Filter (23) fließt, Partikel herauszufiltern,
- in der Fluidführungseinheit (71) angeordnet ist und
- sich dann, wenn die Eingabeeinheit (70) mit dem Grundkörper (9) verbunden ist, zwischen der Ansaugöffnung (AO) und dem Ventil (2, 13) befindet.

2. Analysegerät (100) nach Anspruch 1,
**dadurch gekennzeichnet, dass**
das Ventil (2, 13) einen Ventilkörper (2) und einen Ventilkörper-Sitz (13) umfasst und
das Analysegerät (100) ein mechanisches Verbindungselement (4) umfasst,
wobei dann, wenn das Ventil (2, 13) in der verschließenden Endposition ist, der Ventilkörper (2) am Ventilkörper-Sitz (13) anliegt und dann, wenn das Ventil (2, 13) in der freigebenden Endposition ist, ein Abstand zwischen dem Ventilkörper (2) und dem Ventilkörper-Sitz (13) auftritt, und
wobei das mechanische Verbindungselement (4) den Ventilkörper (2) dergestalt mechanisch mit der Ansaugeinheit (5, 6, 7, 27) verbindet,
dass dann, wenn die Ansaugeinheit (5, 6, 7, 27) eine Gasprobe (Gp) ansaugt und in die Messkammer (3) fördert,
das Ventil (2, 13) aus der einen Endposition in die andere Endposition bewegt wird und
bevorzugt das Ventil (2, 13) aus der freigebenden in die verschließende Endposition bewegt wird.

3. Analysegerät (100) nach einem der vorhergehenden Ansprüche,
**dadurch gekennzeichnet, dass**
das Analysegerät (100) eine Heizung (24) umfasst,
wobei die Heizung (24) dazu ausgestaltet ist, ein Segment (hS) der Fluidführungseinheit (71) zu erwärmen, und
wobei der Filter (23) sich im erwärmten Segment (hS) befindet.

4. Analysegerät (100) nach einem der vorhergehenden Ansprüche,
**dadurch gekennzeichnet, dass**
die Eingabeeinheit (70) sich entlang einer Längsachse (EA) erstreckt und die Fluidführungseinheit (71) sich ebenfalls entlang einer Längsachse (FA) erstreckt,
wobei die beiden Längsachsen (EA, FA) zwischen sich einen Winkel von mindestens 60° einschließen und bevorzugt senkrecht aufeinander stehen.

5. Analysegerät (100) nach einem der vorhergehenden Ansprüche,
**dadurch gekennzeichnet, dass**
der Filter (23) elektrostatisch aufgeladen ist.

6. Analysegerät (100) nach einem der vorhergehenden Ansprüche,
**dadurch gekennzeichnet, dass**
der Filter (23) zwei Schichten umfasst,
wobei die beiden Schichten - gesehen in eine Fließrichtung einer Gasprobe (Gp) durch den Filter (23) hindurch - hintereinander angeordnet sind und jeweils mehrere Öffnungen aufweisen,
wobei die Öffnungen insbesondere Poren sind, die von Fasern der jeweiligen Schicht gebildet werden, und
wobei die Öffnungen dergestalt zueinander versetzt angeordnet sind, dass ein Partikel auf dem Weg durch den Filter (23) hindurch mindestens einmal abgelenkt wird.

7. Analysegerät (100) nach einem der vorhergehenden Ansprüche,
**dadurch gekennzeichnet, dass**
das Analysegerät (100) einen Drucksensor (46) umfasst,
wobei der Drucksensor dazu ausgestaltet ist, den Druck an einer ersten Messposition (MP.1) zu messen,
wobei die erste Messposition (MP.1) sich in der Fluidführungseinheit (71) zwischen dem Filter (23) und der Messkammer (3) oder an oder in der Messkammer (3) oder zwischen der Messkammer (3) und der Ansaugeinheit (5, 6, 7, 27) befindet,
wobei das Analysegerät (100) dazu ausgestaltet ist,
- unter Verwendung eines Signals des Drucksensors (46) den Druck an der ersten Messposition (MP.1) zu messen,
- den Volumenfluss durch den Filter (23) zu messen oder zu ermitteln,
- den aktuellen pneumatischen Widerstand des Filters (23) zu ermitteln und
- dann, wenn der ermittelte pneumatische Widerstand außerhalb eines vorgegebenen Wertebereichs liegt, insbesondere größer als eine vorgegebene obere Schranke ist,
eine entsprechende Meldung zu generieren und in mindestens einer von einem Menschen wahrnehmbaren Form auszugeben,
wobei der pneumatische Widerstand der Quotient aus dem Druckabfall am Filter (23) und dem Volumenfluss durch den Filter (23) ist und
wobei das Analysegerät (100) weiterhin dazu ausgestaltet ist, für die Ermittlung des pneumatischen Widerstands
- den gemessenen Druck an der ersten Messposition (MP.1) und
- den gemessenen oder ermittelten Volumenfluss durch den Filter (23) zu verwenden.

8. Analysegerät (100) nach Anspruch 7,
**dadurch gekennzeichnet, dass**
der Drucksensor (46) zusätzlich dazu ausgestaltet ist, den Druck an einer zweiten Messposition (MP.2) zu messen,
wobei die zweite Messposition (MP.2) sich in der Fluidführungseinheit (71) und bei aufgesetzter Eingabeeinheit (70) zwischen der Ansaugöffnung (AO) und dem Filter (23) befindet und
wobei das Analysegerät (100) dazu ausgestaltet ist,
- unter Verwendung des Signals des Drucksensors (46) den Druck an der zweiten Messposition (MP.2) zu messen und
- den Druckabfall am Filter (23) und / oder den Volumenfluss durch den Filter (23) unter Verwendung der beiden gemessenen Drücke zu ermitteln.

9. Analysegerät (100) nach Anspruch 7 oder Anspruch 8,
**dadurch gekennzeichnet, dass**
der Drucksensor dazu ausgestaltet ist, den Druck an der ersten Messposition (MP.1) wiederholt zu messen, und
das Analysegerät (100) dazu ausgestaltet ist,
- eine Zeitspanne zu ermitteln, in der an der ersten Messposition (MP.1) ein Unterdruck relativ zu einem gemessenen oder anderweitig ermittelten Referenzdruck auftritt,
- unter Verwendung der ermittelten Zeitspanne die Zeitdauer zu ermitteln, die zum Ansaugen der Gasprobe (Gp) verwendet wird,
- das Volumen der angesaugten Gasprobe (Gp) zu ermitteln und
- den Volumenfluss durch den Filter (23) als Quotient aus dem Volumen und der ermittelten Zeitdauer zu ermitteln,
wobei das Analysegerät (100) dazu ausgestaltet ist, den zeitlichen Verlauf des Drucks an der ersten Messposition (MP.1) zu ermitteln und für die Ermittlung der Zeitspanne den zeitlichen Verlauf des Drucks zu verwenden.

10. Analysegerät (100) nach einem der Ansprüche 7 bis 9,
**dadurch gekennzeichnet, dass**
das Analysegerät (100) dazu ausgestaltet ist,
dann, wenn der ermittelte pneumatische Widerstand des Filters (23) kleiner als eine vorgegebene untere Schranke ist,
eine Meldung zu generieren und zu bewirken, dass diese Meldung in mindestens einer von einem Menschen wahrnehmbaren Form ausgegeben wird.

11. Analysegerät (100) nach einem der vorhergehenden Ansprüche,
**dadurch gekennzeichnet, dass**
der Sensor (12) dazu ausgestaltet ist, die Konzentration von Alkohol als der Substanz in der Gasprobe (Gp) in der Messkammer (3) zu messen.

12. Überwachungseinheit zum Überwachen eines Analysegeräts (100) nach einem der Ansprüche 1 bis 6 oder 11,
wobei die Überwachungseinheit einen Drucksensor (46) und eine signalverarbeitende Auswertungseinheit (62) umfasst,
wobei der Drucksensor dazu ausgestaltet ist, den Druck an einer ersten Messposition (MP.1) zu messen,
wobei die erste Messposition (MP.1) sich in der Fluidführungseinheit (71) zwischen dem Filter (23) und der Messkammer (3) oder an oder in der Messkammer (3) oder zwischen der Messkammer (3) und der Ansaugeinheit (5, 6, 7, 27) befindet,
wobei die Auswertungseinheit (62) dazu ausgestaltet ist,
- unter Verwendung eines Signals des Drucksensors (46) den Druck an der ersten Messposition (MP.1) zu ermitteln,
- den Volumenfluss durch den Filter (23) zu ermitteln,
- den aktuellen pneumatischen Widerstand des Filters (23) zu ermitteln und
- dann, wenn der ermittelte pneumatische Widerstand außerhalb eines vorgegebenen Wertebereichs liegt, insbesondere größer als eine vorgegebene obere Schranke ist,
eine entsprechende Meldung zu generieren und zu bewirken, dass die Meldung in mindestens einer von einem Menschen wahrnehmbaren Form ausgegeben wird,
wobei der pneumatische Widerstand der Quotient aus dem Druckabfall am Filter (23) und dem Volumenfluss durch den Filter (23) ist und
wobei die Auswertungseinheit (62) weiterhin dazu ausgestaltet ist, für die Ermittlung des pneumatischen Widerstands
- den ermittelten Druck an der ersten Messposition (MP.1) und
- den gemessenen oder ermittelten Volumenfluss durch den Filter (23) zu verwenden.

13. Überwachungseinheit nach Anspruch 12,
**dadurch gekennzeichnet, dass**
der Drucksensor (46) zusätzlich dazu ausgestaltet ist, den Druck an einer zweiten Messposition (MP.2) zu messen,
wobei die zweite Messposition (MP.2) sich in der Fluidführungseinheit (71) und bei aufgesetzter Eingabeeinheit (70) zwischen der Ansaugöffnung (AO) und dem Filter (23) befindet und
wobei Auswertungseinheit (62) dazu ausgestaltet ist,
- unter Verwendung des Signals des Drucksensors (46) den Druck an der zweiten Messposition (MP.2) zu ermitteln und
- den Druckabfall am Filter (23) und / oder den Volumenfluss durch den Filter (23) unter Verwendung der beiden ermittelten Drücke zu ermitteln.
